# EUROPEAN PATENT APPLICATION

(11) **EP 4 840 858 A1**
(43) Date of publication of application: **18.11.2026**
(21) Application number: 25738483.4
(22) Date of filing: 09.01.2025
(51) Int. Cl.: C07D 471/04, C07D 417/14, C07D 405/14, A61K 31/496, A61K 31/454, A61P 3/10, A61P 3/04, A61P 3/06

(54) **GLP-1R AGONIST PHARMACEUTICAL COMPOSITION AND USE THEREOF**

(30) Priority: 09.01.2024 CN 202410030430; 12.08.2024 CN 202411100857
(71) Applicant: Jiangsu Hengrui Pharmaceuticals Co., Ltd., Lianyungang, Jiangsu 222047 (CN)
(72) Inventor: GUO, Chuanglong, Jinan, Shandong 250104 (CN); WANG, Cong, Jinan, Shandong 250104 (CN); MENG, Lubo, Jinan, Shandong 250104 (CN); LU, Yu, Jinan, Shandong 250104 (CN); ZHU, Ning, Jinan, Shandong 250104 (CN); FAN, Xing, Shanghai 200245 (CN)
(74) Representative: J A Kemp LLP
(86) International application number: PCT/CN2025/071407
(87) International publication number: WO 2025/148954

(57) **Abstract**

Disclosed are a GLP-1R agonist pharmaceutical composition and a use thereof. The pharmaceutical composition comprises a compound represented by formula I, or a pharmaceutically acceptable salt thereof, as an active ingredient. The pharmaceutical composition comprises an acid-resistant compound, and can slowly release active ingredients in the pharmaceutical composition, so as to reduce side effects of in vivo absorption after GLP-1R administration.

## Description

### TECHNICAL FIELD

The present disclosure belongs to the field of pharmaceutical preparations and relates to a GLP-1R agonist pharmaceutical composition and use thereof.

### BACKGROUND ART

Glucagon-like peptide-1 (GLP-1) is an incretin hormone secreted by L-cells in the lower gastrointestinal tract. GLP-1 exerts its effects via binding to its widely distributed specific receptors. Organs confirmed to express GLP-1 receptors include pancreatic islet cells, gastrointestinal tract, lung, brain, kidney, hypothalamus and cardiovascular system, while GLP-1 receptors are putatively present in the liver, adipose tissue and skeletal muscle. GLP-1 not only acts on β cells to promote insulin secretion, but also acts on α cells to inhibit glucagon secretion. There is generally no significant difference in serum GLP-1 levels among patients with normal glucose tolerance, impaired glucose tolerance, and type II diabetes mellitus. Nevertheless, β-cells exhibit impaired responsiveness to GLP-1 after food intake, and such responsiveness can be markedly improved upon continuous GLP-1 infusion under certain conditions. Endogenous human GLP-1 features an extremely short duration of action (intravenous half-life t1/2<1.5 min), which renders endogenous GLP-1 unsuitable for clinical treatment of diabetes.

Peptidic GLP-1 receptor agonists (e.g., liraglutide, exenatide) are capable of reducing fasting and postprandial blood glucose and improving glycemic control in patients with type II diabetes mellitus. However, peptidic GLP-1 agents suffer from poor oral bioavailability and unsatisfactory patient compliance due to parenteral administration; accordingly, there is an urgent need to develop small-molecule GLP-1 receptor agonists with favorable oral bioavailability.

Published patent applications for small-molecule GLP-1 receptor agonists include WO2009111700A2, WO2010114824A1, WO2018109607A1, WO2019239319A1 and WO2018056453A1, etc.

### SUMMARY

Disclosed is a pharmaceutical composition comprising a compound represented by formula I or a pharmaceutically acceptable salt thereof as an active ingredient, 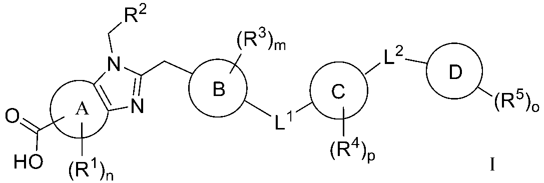 wherein,
ring A is selected from phenyl or 5- or 6-membered heteroaryl;
each R¹ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
R² is selected from 3- to 6-membered heterocycloalkyl or 5- or 6-membered heteroaryl, wherein the heterocycloalkyl or heteroaryl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
ring B is selected from 3- to 6-membered heterocycloalkyl, phenyl, or 5- or 6-membered heteroaryl;
each R³ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
or any two R³ groups, together with the adjacent atoms to which they are attached, form C₃₋₆ cycloalkyl or 3- to 6-membered heterocycloalkyl, wherein the cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
L¹ is selected from -O-, -S-, -C(O)O-, -OC(O)-, -C(O)-, -S(O)-, -S(O)₂-, -C(R^{1a}R^{1b})-, -OC(R^{1a}R^{1b})-, -C(R^{1a}R^{1b})O-, -C(O)N(R^{1c})-, -N(R^{1c})(O)C-, -S(O)₂N(R^{1d})-, -N(R^{1e})- or a bond,
each of R^{1a}, R^{1b}, R^{1c}, R^{1d} and R^{1e} is independently selected from hydrogen, halogen, hydroxy, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl or C₁₋₆ alkoxy;
ring C is selected from C₆₋₁₀ aryl or 5- or 10-membered heteroaryl;
each R⁴ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
L² is selected from-O-, -S-, -C(O)O-, -OC(O)-, -C(O)-, -S(O)-, -S(O)₂-, -C(R^{2a}R^{2b})-, -OC(R^{2a}R^{2b})-, -C(R^{2a}R^{2b})O-, - C(O)N(R^{2c})-, -N(R^{2c})(O)C-, -S(O)₂N(R^{2d})-, -N(R^{2e})- or a bond;
each of R^{2a}, R^{2b}, R^{2c}, R^{2d} and R^{2e} is independently selected from hydrogen, halogen, hydroxy, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl or C₁₋₆ alkoxy;
ring D is selected from C₆₋₁₀ aryl or 5- or 10-membered heteroaryl;
each R⁵ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
n is selected from 0, 1, 2, 3 or 4;
m is selected from 0, 1, 2, 3 or 4;
o is selected from 0, 1, 2, 3 or 4;
p is selected from 0, 1, 2, 3 or 4.

In some embodiments, the pharmaceutical composition further comprises an acid-resistant compound. The acid-resistant compound refers to any pharmaceutically acceptable compound capable of neutralizing acids. In some embodiments, the acid-resistant compound is a water-soluble acid-resistant compound, including but not limited to sodium carbonate, sodium bicarbonate, potassium carbonate, and potassium bicarbonate.

In other embodiments, the acid-resistant compound in the pharmaceutical composition is sodium carbonate.

In another aspect, the pharmaceutical composition disclosed herein further comprises pharmaceutically acceptable excipients, which are known or readily determinable by those skilled in the art and are selected from at least one of disintegrants, fillers, binders and lubricants, without limitation.

Fillers provide bulk to bring tablets to a practically processable size; they may also facilitate manufacturing processes and improve physical properties of solid formulations such as flowability, compressibility and hardness. The fillers described in the present disclosure are known or readily identifiable by those skilled in the art and are selected from at least one of lactose, sucrose, starch, pregelatinized starch, mannitol, dibasic calcium phosphate and microcrystalline cellulose, without limitation. In some embodiments, the filler accounts for 20-95% by weight of the pharmaceutical composition, and can be 20%, 22%, 25%, 28%, 30%, 32%, 35%, 38%, 40%, 42%, 45%, 47%, 50%, 52%, 55%, 58%, 60%, 62%, 65%, 68%, 70%, 72%, 75%, 78%, 80%, 82%, 85%, 88%, 90%, 92%, or 95%. In some embodiments, the filler accounts for 20-80% by weight of the pharmaceutical composition. In some embodiments, the filler accounts for 60-80% by weight of the pharmaceutical composition. In some embodiments, the filler accounts for 50-70% by weight of the pharmaceutical composition.

The disintegrants described in the present disclosure are known or identifiable by those skilled in the art and are selected from at least one of croscarmellose sodium, crospovidone, sodium starch glycolate and alginic acid, without limitation. In some embodiments, the disintegrant accounts for 1-20% by weight of the pharmaceutical composition, and can be 1.0%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17%, 18%, 19%, or 20%. In some embodiments, the disintegrant preferably accounts for 5-15% by weight of the pharmaceutical composition. In some embodiments, the disintegrant preferably accounts for 5-10% by weight of the pharmaceutical composition. In some embodiments, the disintegrant preferably accounts for 1-5% by weight of the pharmaceutical composition.

The binders described in the present disclosure are known or identifiable by those skilled in the art and are selected from at least one of copovidone, povidone, starch, methylcellulose, carboxymethylcellulose, hydroxypropyl cellulose, hydroxypropyl methylcellulose or low-substituted hydroxypropyl cellulose, without limitation. In some embodiments, the binder accounts for 0.5-10% by weight of the pharmaceutical composition, and can be 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10%. In some embodiments, the binder accounts for 0.5-5% by weight of the pharmaceutical composition. In some embodiments, the binder accounts for 1-5% by weight of the pharmaceutical composition. In some embodiments, the binder accounts for 1-4% by weight of the pharmaceutical composition.

The lubricants described in the present disclosure are known or identifiable by those skilled in the art and are selected from at least one of magnesium stearate, stearic acid, palmitic acid, talc and sodium stearyl fumarate, without limitation. In some embodiments, the lubricant accounts for 0.1-5% by weight of the pharmaceutical composition, and can be 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, or 5%. In some embodiments, the lubricant accounts for 0.1-2% by weight of the pharmaceutical composition. In another aspect, ring D in the compound represented by formula I or a pharmaceutically acceptable salt thereof according to the present disclosure is selected from phenyl.

In some embodiments, ring B in the compound represented by formula I or a pharmaceutically acceptable salt thereof is selected from 3- to 6-membered heterocycloalkyl, including 3-membered heterocycloalkyl, 4-membered heterocycloalkyl, 5-membered heterocycloalkyl or 6-membered heterocycloalkyl. In some embodiments, ring B in the compound represented by formula I or a pharmaceutically acceptable salt thereof is selected from 6-membered heterocycloalkyl, for example:

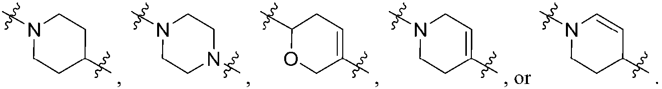

In other embodiments, R² in the compound represented by formula I or a pharmaceutically acceptable salt thereof is selected from 4- to 5-membered heterocycloalkyl or 5-membered heteroaryl, such as

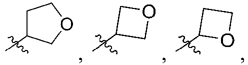

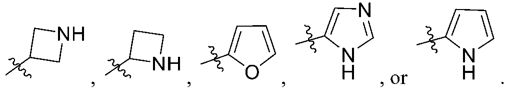

In other embodiments, R² in the compound represented by formula I or a pharmaceutically acceptable salt thereof is selected from

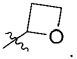

In other embodiments, R² in the compound represented by formula I or a pharmaceutically acceptable salt thereof is selected from

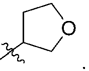

In other embodiments, R² in the compound represented by formula I or a pharmaceutically acceptable salt thereof is selected from

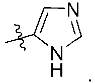

In some embodiments, the compound represented by formula I or a pharmaceutically acceptable salt thereof is a compound represented by formula II-1 or a pharmaceutically acceptable salt thereof, 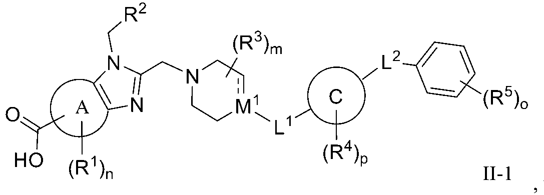 wherein M¹ is an N atom or a C atom; - - - - - is a single bond or a double bond; when M is an N atom, - - - - - is a single bond; when M is a C atom, - - - - - is a single bond or a double bond; ring A, ring C, R¹, R², R³, R⁴, R⁵, L¹, L², n, m, o and p are as defined for the compound represented by formula I.

Furthermore, in some embodiments, ring A in the compound represented by formula I or formula II-1 or a pharmaceutically acceptable salt thereof is selected from phenyl or 5-membered heteroaryl, such as

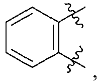

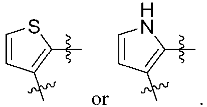

In other embodiments, each R¹ in the compound represented by formula I or formula II-1 or a pharmaceutically acceptable salt thereof is the same or different and is independently selected from halogens (e.g., -F or -Br), hydroxy or cyano.

In other embodiments, each R¹ in the compound represented by formula I or formula II-1 or a pharmaceutically acceptable salt thereof is the same or different and is independently selected from halogen, C₁₋₆ alkyl, C₁₋₆ alkoxy or C₃₋₆ cycloalkyl, wherein the alkyl, alkoxy or cycloalkyl is optionally substituted with one or more groups selected from halogen, hydroxy, cyano, nitro, amino or C₁₋₆ alkyl.

In some embodiments, each R³ in the compound represented by formula I or formula II-1 or a pharmaceutically acceptable salt thereof is the same or different and is independently selected from halogens (e.g., -F or -Br), hydroxy or cyano.

In some embodiments, each R³ in the compound represented by formula I or formula II-1 or a pharmaceutically acceptable salt thereof is the same or different and is independently selected from halogen, C₁₋₆ alkyl, C₁₋₆ alkoxy or C₃₋₆ cycloalkyl, wherein the alkyl, alkoxy or cycloalkyl is optionally substituted with one or more groups selected from halogen, hydroxy, cyano, nitro, amino or C₁₋₆ alkyl.

In other embodiments, any two R³ groups in the compound represented by formula I or formula II-1 or a pharmaceutically acceptable salt thereof, together with the adjacent atoms to which they are attached, form C₃₋₄ cycloalkyl (e.g., cyclopropyl) or 3- to 5-membered heterocycloalkyl (e.g., tetrahydrofuranyl), wherein the cycloalkyl or heterocycloalkyl is optionally substituted with one or more groups selected from halogen, hydroxy, cyano, nitro, amino or C₁₋₆ alkyl.

Furthermore, in some embodiments, ring C in the compound represented by formula I or formula II-1 or a pharmaceutically acceptable salt thereof is selected from

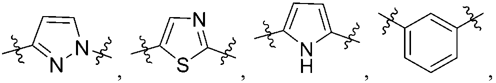

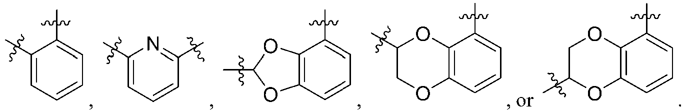

In some embodiments, the compound represented by formula I or a pharmaceutically acceptable salt thereof is a compound represented by formula II-1a or a pharmaceutically acceptable salt thereof 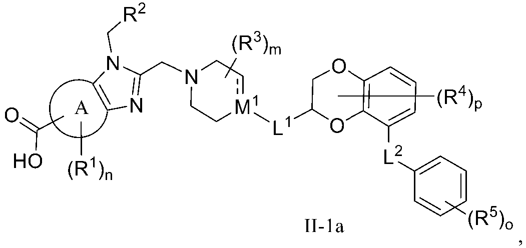 wherein ring A, R¹, R², R³, R⁴, R⁵, L¹, L², n, m, o and p are as defined for the compound represented by formula I; M¹ is an N atom or a C atom; - - - - - is a single bond or a double bond; when M is an N atom, - - - - - is a single bond; when M is a C atom, - - - - - is a single bond or a double bond.

In some embodiments, ring A in the compound represented by formula II-1a or a pharmaceutically acceptable salt thereof is selected from phenyl or 5-membered heteroaryl, such as

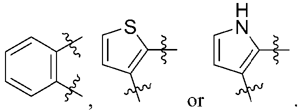

In some embodiments, the compound represented by formula II-1a or a pharmaceutically acceptable salt thereof is a compound represented by formula II-1a-a or a pharmaceutically acceptable salt thereof 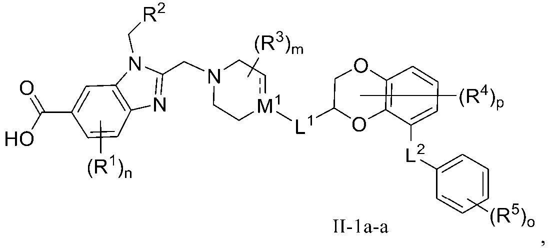 wherein R¹, R², R³, R⁴, R⁵, L¹, L², n, m, o, p and M¹ are as defined for the compound represented by formula II-1a; - - - - - is a single bond or a double bond; when M is an N atom, - - - - - is a single bond; when M is a C atom, - - - - - is a single bond or a double bond.

In some embodiments, in the compound represented by formula I or formula II-1a or formula II-1a-a or a pharmaceutically acceptable salt thereof, L¹ is selected from a bond, and L² is selected from a bond.

In some embodiments, in the compound represented by formula I or formula II-1a or formula II-1a-a or a pharmaceutically acceptable salt thereof, L¹ is selected from -O-, L² is selected from -OC(R^{2a}R^{2b})- or -C(R^{2a}R^{2b})O-, and R^{2a} and R^{2b} are as defined above.

In some embodiments, in the compound represented by formula I or formula II-1a or formula II-1a-a or a pharmaceutically acceptable salt thereof, L¹ is selected from a bond, L² is selected from -OC(R^{2a}R^{2b})- or -C(R^{2a}R^{2b})O-, and R^{2a} and R^{2b} are as defined above.

In some embodiments, each of R^{2a} and R^{2b} in the compound represented by formula I, formula II-1a or formula II-1a-a or a pharmaceutically acceptable salt thereof is independently selected from hydrogen, halogen, hydroxy or C₁₋₆ alkyl, wherein the alkyl is optionally substituted with one or more groups selected from halogen, hydroxy, cyano, nitro or amino.

In some embodiments, each of R^{2a} and R^{2b} in the compound represented by formula I or formula II-1a or formula II-1a-a or a pharmaceutically acceptable salt thereof is selected from hydrogen.

In some embodiments, in the compound represented by formula I or formula II-1a or formula II-1a-a or a pharmaceutically acceptable salt thereof, L¹ is selected from a bond, and L² is selected from -OCH₂- or -CH₂O-.

In some embodiments, in the compound represented by formula I or formula II-1a or formula II-1a-a or a pharmaceutically acceptable salt thereof, L¹ is selected from -O-, and L² is selected from -OCH₂- or -CH₂O-.

In some embodiments, the compound represented by formula I or a pharmaceutically acceptable salt thereof is a compound represented by formula II-1b or a pharmaceutically acceptable salt thereof 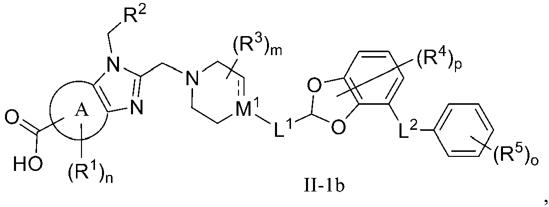 wherein ring A, R¹, R², R³, R⁴, R⁵, L¹, L², n, m, o and p are as defined for the compound represented by formula I; M¹ is an N atom or a C atom; - - - - - is a single bond or a double bond; when M is an N atom, - - - - - is a single bond; when M is a C atom, - - - - - is a single bond or a double bond.

In some embodiments, ring A in the compound represented by formula II-1b or a pharmaceutically acceptable salt thereof is selected from phenyl or 5-membered heteroaryl, such as

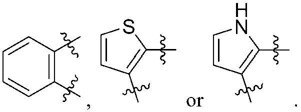

In some embodiments, the compound represented by formula II-1b or a pharmaceutically acceptable salt thereof is a compound represented by formula II-1b-a or a pharmaceutically acceptable salt thereof 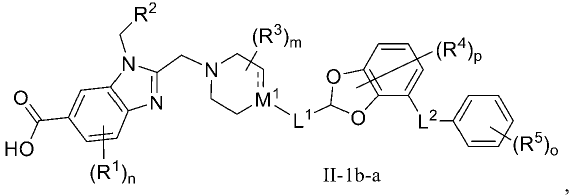 wherein R¹, R², R³, R⁴, R⁵, L¹, L², n, m, o, p and M¹ are as defined for the compound represented by formula II-1b; - - - - - is a single bond or a double bond; when M is an N atom, - - - - - is a single bond; when M is a C atom, - - - - - is a single bond or a double bond.

In some embodiments, in the compound represented by formula II-1b or formula II-1b-a or a pharmaceutically acceptable salt thereof, L¹ is selected from a bond, and L² is selected from a bond.

In some embodiments, in the compound represented by formula II-1b or II-1b-a or a pharmaceutically acceptable salt thereof, L¹ is selected from -O-, L² is selected from -OC(R^{2a}R^{2b})- or -C(R^{2a}R^{2b})O-, and R^{2a} and R^{2b} are as defined above.

In some embodiments, in the compound represented by formula II-1b or II-1b-a or a pharmaceutically acceptable salt thereof, L¹ is selected from a bond, L² is selected from -OC(R^{2a}R^{2b})- or -C(R^{2a}R^{2b})O-, and R^{2a} and R^{2b} are as defined above.

In some embodiments, each of R^{2a} and R^{2b} in the compound represented by formula II-1b or formula II-1b-a or a pharmaceutically acceptable salt thereof is independently selected from hydrogen, halogen, hydroxy or C₁₋₆ alkyl, wherein the alkyl is optionally substituted with one or more groups selected from halogen, hydroxy, cyano, nitro or amino.

In some embodiments, each of R^{2a} and R^{2b} in the compound represented by formula II-1b or formula II-1b-a or a pharmaceutically acceptable salt thereof is selected from hydrogen.

In some embodiments, in the compound represented by formula II-1b or formula II-1b-a or a pharmaceutically acceptable salt thereof, L¹ is selected from -O-, and L² is selected from -OCH₂- or -CH₂O-.

In some embodiments, in the compound represented by formula II-1b or II-1b-a or a pharmaceutically acceptable salt thereof, L¹ is selected from a bond, and L² is selected from -OCH₂- or -CH₂O-.

In some embodiments, the compound represented by formula I or a pharmaceutically acceptable salt thereof is a compound represented by formula II-1c or a pharmaceutically acceptable salt thereof 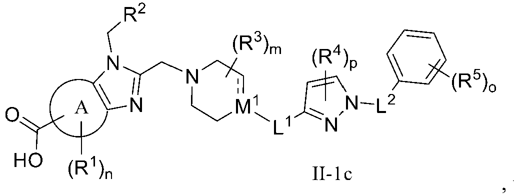 wherein ring A, R¹, R², R³, R⁴, R⁵, L¹, L², n, m, o and p are as defined for the compound represented by formula I; M¹ is an N atom or a C atom; - - - - - is a single bond or a double bond; when M is an N atom, - - - - - is a single bond; when M is a C atom, - - - - - is a single bond or a double bond.

In some embodiments, ring A in the compound represented by formula II-1c or a pharmaceutically acceptable salt thereof is selected from phenyl or 5-membered heteroaryl, such as

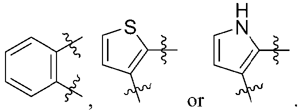

In some embodiments, the compound represented by formula II-1c or a pharmaceutically acceptable salt thereof is a compound represented by formula II-1c-a or a pharmaceutically acceptable salt thereof 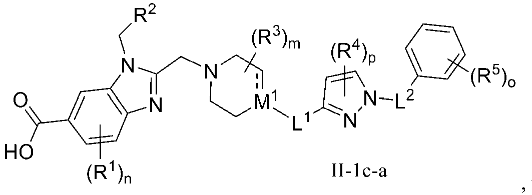 wherein R¹, R², R³, R⁴, R⁵, L¹, L², n, m, o, p and M¹ are as defined for the compound represented by formula II-1c; - - - - - is a single bond or a double bond; when M is an N atom, - - - - - is a single bond; when M is a C atom, - - - - - is a single bond or a double bond.

In some embodiments, in the compound represented by formula II-1c or formula II-1c-a or a pharmaceutically acceptable salt thereof, L¹ is selected from a bond, and L² is selected from a bond.

In some embodiments, in the compound represented by formula II-1c or formula II-1c-a or a pharmaceutically acceptable salt thereof, L¹ is selected from -O-, L² is selected from -OC(R^{2a}R^{2b})- or -C(R^{2a}R^{2b})O-, and R^{2a} and R^{2b} are as defined above.

In some embodiments, in the compound represented by formula II-1c or formula II-1c-a or a pharmaceutically acceptable salt thereof, L¹ is selected from a bond, L² is selected from -OC(R^{2a}R^{2b})- or -C(R^{2a}R^{2b})O-, and R^{2a} and R^{2b} are as defined above.

In some embodiments, each of R^{2a} and R^{2b} in the compound represented by formula II-1c or formula II-1c-a or a pharmaceutically acceptable salt thereof is independently selected from hydrogen, halogen, hydroxy or C₁₋₆ alkyl, wherein the alkyl is optionally substituted with one or more groups selected from halogen, hydroxy, cyano, nitro or amino.

In some embodiments, each of R^{2a} and R^{2b} in the compound represented by formula II-1c or formula II-1c-a or a pharmaceutically acceptable salt thereof is selected from hydrogen.

In some embodiments, in the compound represented by formula II-1c or formula II-1c-a or a pharmaceutically acceptable salt thereof, L¹ is selected from -O-, and L² is selected from -OCH₂- or -CH₂O-.

In some embodiments, in the compound represented by formula II-1c or formula II-1c-a or a pharmaceutically acceptable salt thereof, L¹ is selected from a bond, and L² is selected from -OCH₂- or -CH₂O-.

In some embodiments, the compound represented by formula I or a pharmaceutically acceptable salt thereof is a compound represented by formula II-1d or a pharmaceutically acceptable salt thereof 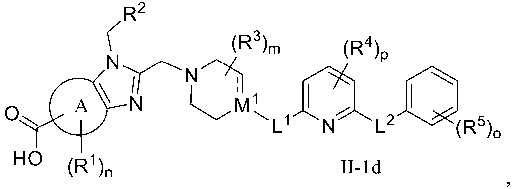 wherein ring A, R¹, R², R³, R⁴, R⁵, L¹, L², n, m, o and p are as defined for the compound represented by formula I; M¹ is an N atom or a C atom; - - - - - is a single bond or a double bond; when M is an N atom, - - - - - is a single bond; when M is a C atom, - - - - - is a single bond or a double bond.

In some embodiments, ring A in the compound represented by formula II-1d or a pharmaceutically acceptable salt thereof is selected from phenyl or 5-membered heteroaryl, such as

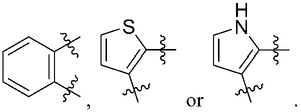

In some embodiments, the compound represented by formula II-1d or a pharmaceutically acceptable salt thereof is a compound represented by formula II-1d-a or a pharmaceutically acceptable salt thereof 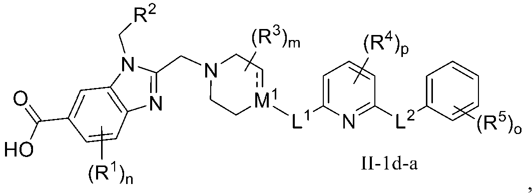 wherein R¹, R², R³, R⁴, R⁵, L¹, L², n, m, o, p and M¹ are as defined for the compound represented by formula II-1d; - - - - - is a single bond or a double bond; when M is an N atom, - - - - - is a single bond; when M is a C atom, - - - - - is a single bond or a double bond.

In some embodiments, in the compound represented by formula II-1d or formula II-1d-a or a pharmaceutically acceptable salt thereof, L¹ is selected from a bond, and L² is selected from a bond.

In some embodiments, in the compound represented by formula II-1d or formula II-1d-a or a pharmaceutically acceptable salt thereof, L¹ is selected from -O-, L² is selected from -OC(R^{2a}R^{2b})- or -C(R^{2a}R^{2b})O-, and R^{2a} and R^{2b} are as defined above.

In some embodiments, in the compound represented by formula II-1d or formula II-1d-a or a pharmaceutically acceptable salt thereof, L¹ is selected from a bond, L² is selected from -OC(R^{2a}R^{2b})- or -C(R^{2a}R^{2b})O-, and R^{2a} and R^{2b} are as defined above.

In some embodiments, each of R^{2a} and R^{2b} in the compound represented by formula II-1d or formula II-1d-a or a pharmaceutically acceptable salt thereof is independently selected from hydrogen, halogen, hydroxy or C₁₋₆ alkyl, wherein the alkyl is optionally substituted with one or more groups selected from halogen, hydroxy, cyano, nitro or amino.

In some embodiments, each of R^{2a} and R^{2b} in the compound represented by formula II-1d or formula II-1d-a or a pharmaceutically acceptable salt thereof is selected from hydrogen.

In some embodiments, in the compound represented by formula II-1d or formula II-1d-a or a pharmaceutically acceptable salt thereof, L¹ is selected from -O-, and L² is selected from -OCH₂- or -CH₂O-.

In some embodiments, in the compound represented by formula II-1d or formula II-1d-a or a pharmaceutically acceptable salt thereof, L¹ is selected from a bond, and L² is selected from -OCH₂- or -CH₂O-.

In another aspect, ring D in the compound represented by formula I or a pharmaceutically acceptable salt thereof according to the present disclosure is selected from 5- or 10-membered heteroaryl, such as

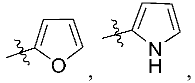

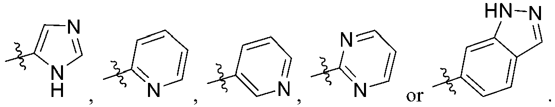

In other embodiments, ring D in the compound represented by formula I or a pharmaceutically acceptable salt thereof is selected from

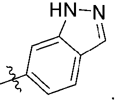

In other embodiments, ring D in the compound represented by formula I or a pharmaceutically acceptable salt thereof is selected from

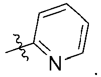

In some embodiments, the compound represented by formula I or a pharmaceutically acceptable salt thereof is a compound represented by formula II-1e or a pharmaceutically acceptable salt thereof 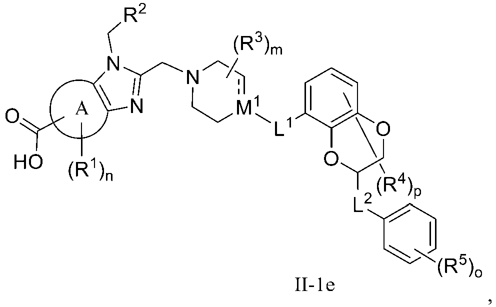 wherein ring A, R¹, R², R³, R⁴, R⁵, L¹, L², n, m, o and p are as defined for the compound represented by formula I; M¹ is an N atom or a C atom; - - - - - is a single bond or a double bond; when M is an N atom, - - - - - is a single bond; when M is a C atom, - - - - - is a single bond or a double bond.

In some embodiments, ring A in the compound represented by formula II-1e or a pharmaceutically acceptable salt thereof is selected from phenyl or 5-membered heteroaryl, such as

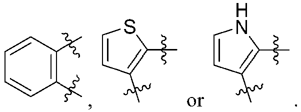

In some embodiments, the compound represented by formula II-1e or a pharmaceutically acceptable salt thereof is a compound represented by formula II-1e-a or a pharmaceutically acceptable salt thereof 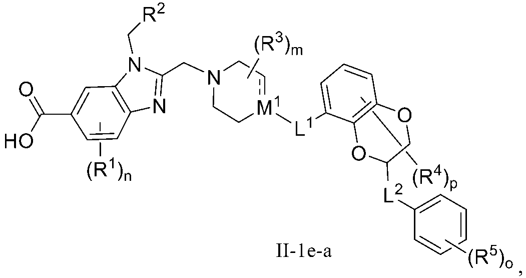 wherein R¹, R², R³, R⁴, R⁵, L¹, L², n, m, o, p and M¹ are as defined for the compound represented by formula II-1e; - - - - - is a single bond or a double bond; when M is an N atom, - - - - - is a single bond; when M is a C atom, - - - - - is a single bond or a double bond.

In some embodiments, in the compound represented by formula I or formula II-1e or formula II-1e-a or a pharmaceutically acceptable salt thereof, L¹ is selected from a bond, and L² is selected from a bond.

In some embodiments, in the compound represented by formula I or formula II-1e or formula II-1e-a or a pharmaceutically acceptable salt thereof, L¹ is selected from -O-, L² is selected from -OC(R^{2a}R^{2b})- or -C(R^{2a}R^{2b})O-, and R^{2a} and R^{2b} are as defined above.

In some embodiments, in the compound represented by formula I or formula II-1e or formula II-1e-a or a pharmaceutically acceptable salt thereof, L¹ is selected from a bond, L² is selected from -OC(R^{2a}R^{2b})- or -C(R^{2a}R^{2b})O-, and R^{2a} and R^{2b} are as defined above.

In some embodiments, each of R^{2a} and R^{2b} in the compound represented by formula I, formula II-1e or formula II-1e-a or a pharmaceutically acceptable salt thereof is independently selected from hydrogen, halogen, hydroxy or C₁₋₆ alkyl, wherein the alkyl is optionally substituted with one or more groups selected from halogen, hydroxy, cyano, nitro or amino.

In some embodiments, each of R^{2a} and R^{2b} in the compound represented by formula I or formula II-1e or formula II-1e-a or a pharmaceutically acceptable salt thereof is selected from hydrogen.

In some embodiments, in the compound represented by formula I or formula II-1e or formula II-1e-a or a pharmaceutically acceptable salt thereof, L¹ is selected from a bond, and L² is selected from -OCH₂- or -CH₂O-.

In some embodiments, in the compound represented by formula I or formula II-1e or formula II-1e-a or a pharmaceutically acceptable salt thereof, L¹ is selected from -O-, and L² is selected from -OCH₂- or -CH₂O-.

In some embodiments, the compound represented by formula I or a pharmaceutically acceptable salt thereof is a compound represented by formula II-1f or a pharmaceutically acceptable salt thereof 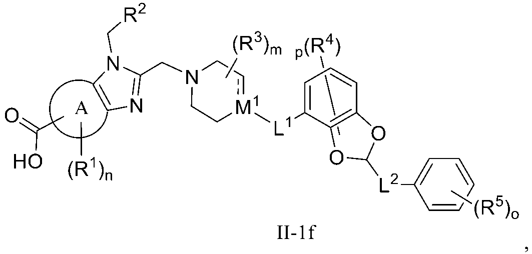 wherein ring A, R¹, R², R³, R⁴, R⁵, L¹, L², n, m, o and p are as defined for the compound represented by formula I; M¹ is an N atom or a C atom; - - - - - is a single bond or a double bond; when M is an N atom, - - - - - is a single bond; when M is a C atom, - - - - - is a single bond or a double bond.

In some embodiments, ring A in the compound represented by formula II-1f or a pharmaceutically acceptable salt thereof is selected from phenyl or 5-membered heteroaryl, such as

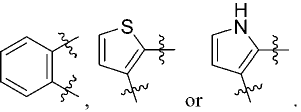

In some embodiments, the compound represented by formula II-1f or a pharmaceutically acceptable salt thereof is a compound represented by formula II-1f-a or a pharmaceutically acceptable salt thereof 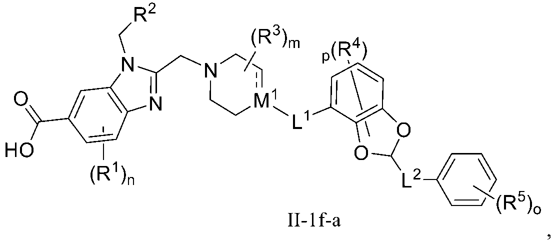 **wherein** R¹, R², R³, R⁴, R⁵, L¹, L², n, m, o, p and M¹ are as defined for the compound represented by formula II-1f; - - - - - is a single bond or a double bond; when M is an N atom, - - - - - is a single bond; when M is a C atom, - - - - - is a single bond or a double bond.

In some embodiments, in the compound represented by formula II-1f or formula II-1f-a or a pharmaceutically acceptable salt thereof, L¹is selected from a bond, and L² is selected from a bond.

In some embodiments, in the compound represented by formula II-1f or formula II-1f-a or a pharmaceutically acceptable salt thereof, L¹ is selected from -O-, L² is selected from -OC(R^{2a}R^{2b})- or -C(R^{2a}R^{2b})O-, and R^{2a} and R^{2b} are as defined above.

In some embodiments, in the compound represented by formula II-1f or formula II-1f-a or a pharmaceutically acceptable salt thereof, L¹ is selected from a bond, L² is selected from -OC(R^{2a}R^{2b})- or -C(R^{2a}R^{2b})O-, and R^{2a} and R^{2b} are as defined above.

In some embodiments, each of R^{2a} and R^{2b} in the compound represented by formula II-1f or formula II-1f-a or a pharmaceutically acceptable salt thereof is independently selected from hydrogen, halogen, hydroxy or C₁₋₆ alkyl, wherein the alkyl is optionally substituted with one or more groups selected from halogen, hydroxy, cyano, nitro or amino.

In some embodiments, each of R^{2a} and R^{2b} in the compound represented by formula II-1f or formula II-1f-a or a pharmaceutically acceptable salt thereof is selected from hydrogen.

In some embodiments, in the compound represented by formula II-1f or formula II-1f-a or a pharmaceutically acceptable salt thereof, L¹ is selected from -O-, and L² is selected from -OCH₂- or -CH₂O-.

In some embodiments, in the compound represented by formula II-1f or formula II-1f-a or a pharmaceutically acceptable salt thereof, L¹ is selected from a bond, and L² is selected from -OCH₂- or -CH₂O-.

In some embodiments, the compound represented by formula I or a pharmaceutically acceptable salt thereof is a compound represented by formula III-1b or a pharmaceutically acceptable salt thereof 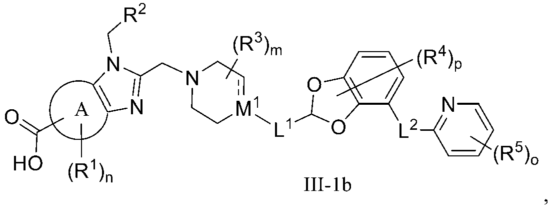 wherein ring A, R¹, R², R³, R⁴, R⁵, L¹, L², n, m, o and p are as defined for the compound represented by formula I; M¹ is an N atom or a C atom; - - - - - is a single bond or a double bond; when M is an N atom, - - - - - is a single bond; when M is a C atom, - - - - - is a single bond or a double bond.

In some embodiments, ring A in the compound represented by formula III-1b or a pharmaceutically acceptable salt thereof is selected from phenyl or 5-membered heteroaryl, such as

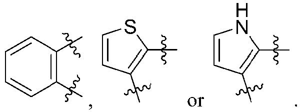

In some embodiments, ring A in the compound represented by formula III-1b or a pharmaceutically acceptable salt thereof is selected from

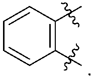

In some embodiments, the compound represented by formula III-1b or a pharmaceutically acceptable salt thereof is a compound represented by formula III-1b-a or a pharmaceutically acceptable salt thereof 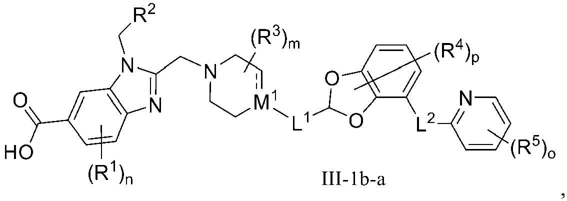 wherein R¹, R², R³, R⁴, R⁵, L¹, L², n, m, o, p and M¹ are as defined for the compound represented by formula III-1b; - - - - - is a single bond or a double bond; when M is an N atom, - - - - - is a single bond; when M is a C atom, - - - - - is a single bond or a double bond.

In some embodiments, in the compound represented by formula III-1b or formula III-1b-a-a or a pharmaceutically acceptable salt thereof, L¹ is selected from a bond, and L² is selected from a bond.

In some embodiments, in the compound represented by formula III-1b or formula III-1b-a or a pharmaceutically acceptable salt thereof, L¹ is selected from -O-, and L² is selected from -OCH₂- or -CH₂O-.

In some embodiments, in the compound represented by formula III-1b or formula III-1b-a or a pharmaceutically acceptable salt thereof, L¹ is selected from a bond, and L² is selected from -OCH₂- or -CH₂O-.

In some embodiments, the compound represented by formula I or a pharmaceutically acceptable salt thereof is a compound represented by formula III-1d or a pharmaceutically acceptable salt thereof 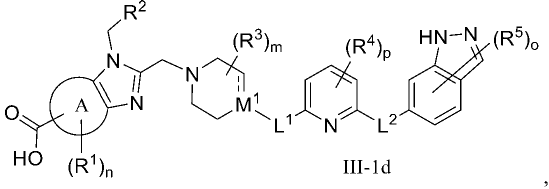 wherein ring A, R¹, R², R³, R⁴, R⁵, L¹, L², n, m, o and p are as defined for the compound represented by formula I; M¹ is an N atom or a C atom; - - - - - is a single bond or a double bond; when M is an N atom, - - - - - is a single bond; when M is a C atom, - - - - - is a single bond or a double bond.

In some embodiments, ring A in the compound represented by formula III-1d or a pharmaceutically acceptable salt thereof is selected from phenyl or 5-membered heteroaryl, such as

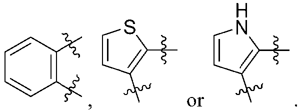

In some embodiments, ring A in the compound represented by formula III-1d or a pharmaceutically acceptable salt thereof is selected from

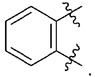

In some embodiments, the compound represented by formula III-1d or a pharmaceutically acceptable salt thereof is a compound represented by formula III-1d-a or a pharmaceutically acceptable salt thereof 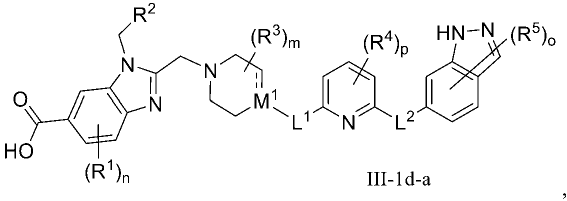 wherein R¹, R², R³, R⁴, R⁵, L¹, L², n, m, o, p and M¹ are as defined for the compound represented by formula III-1b; - - - - - is a single bond or a double bond; when M is an N atom, - - - - - is a single bond; when M is a C atom, - - - - - is a single bond or a double bond.

In some embodiments, in the compound represented by formula III-1d or formula III-1d-a or a pharmaceutically acceptable salt thereof, L¹ is selected from a bond, and L² is selected from a bond.

In some embodiments, in the compound represented by formula III-1d or formula III-1d-a or a pharmaceutically acceptable salt thereof, L¹ is selected from -O-, and L² is selected from -OCH₂- or -CH₂O-.

In some embodiments, in the compound represented by formula III-1d or formula III-1d-a or a pharmaceutically acceptable salt thereof, L¹ is selected from a bond, and L² is selected from -OCH₂- or -CH₂O-.

In another aspect, in some embodiments, each R¹ in the compound represented by formula I or a pharmaceutically acceptable salt thereof is the same or different and is independently selected from halogen, cyano, C₁₋₆ alkyl or C₁₋₆ alkoxy, wherein the alkyl or alkoxy is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl.

In some embodiments, each R¹ in the compound represented by formula I or a pharmaceutically acceptable salt thereof is the same or different and is independently selected from halogen or C₁₋₆ alkoxy, such as fluorine, methoxy or ethoxy.

In some embodiments, each R¹ in the compound represented by formula I or a pharmaceutically acceptable salt thereof is the same or different and is independently selected from halogen or C₁₋₆ alkyl, such as fluorine, methyl or ethyl.

In some embodiments, each R¹ in the compound represented by formula I or a pharmaceutically acceptable salt thereof is the same or different and is independently selected from halogen, cyano, C₃₋₆ cycloalkyl or 3- to 6-membered heterocycloalkyl, wherein the cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl.

In some embodiments, each R¹ in the compound represented by formula I or a pharmaceutically acceptable salt thereof is the same or different and is independently selected from halogen or C₁₋₆ cycloalkyl, such as fluorine, cyclopropyl or cyclobutyl.

In some embodiments, each R⁵ in the compound represented by formula I or a pharmaceutically acceptable salt thereof is the same or different and is independently selected from halogen, cyano, C₁₋₆ alkyl or 3- to 6-membered heterocycloalkyl, wherein the alkyl or heterocycloalkyl is optionally substituted with one or more groups selected from halogen, hydroxy, cyano, nitro, amino, C₁₋₆ alkyl or C₁₋₆ alkoxy.

In some embodiments, each R⁵ in the compound represented by formula I or a pharmaceutically acceptable salt thereof is the same or different and is independently selected from halogen, cyano, C₁₋₆ alkyl or C₁₋₆ alkoxy, wherein the alkyl or alkoxy is optionally substituted with one or more groups selected from halogen, hydroxy, cyano, nitro, amino, C₁₋₆ alkyl or C₁₋₆ alkoxy.

In some embodiments, each R⁵ in the compound represented by formula I or a pharmaceutically acceptable salt thereof is the same or different and is independently selected from halogen, cyano, C₁₋₆ alkyl or 3- to 6-membered heterocycloalkyl, such as fluorine, chlorine, cyano, methyl, ethyl, oxetanyl or tetrahydrofuranyl.

In some embodiments, each R⁴ in the compound represented by formula I or a pharmaceutically acceptable salt thereof is the same or different and is independently selected from halogen, cyano, C₁₋₆ alkyl or C₁₋₆ alkoxy, wherein the alkyl or alkoxy is optionally substituted with one or more groups selected from halogen, hydroxy, cyano, nitro, amino, C₁₋₆ alkyl or C₁₋₆ alkoxy.

In some embodiments, each R⁴ in the compound represented by formula I or a pharmaceutically acceptable salt thereof is the same or different and is independently selected from halogen, cyano or C₁₋₆ alkyl, such as fluorine, chlorine, cyano, methyl or ethyl.

The compound represented by formula I disclosed herein or a pharmaceutically acceptable salt thereof is selected from:

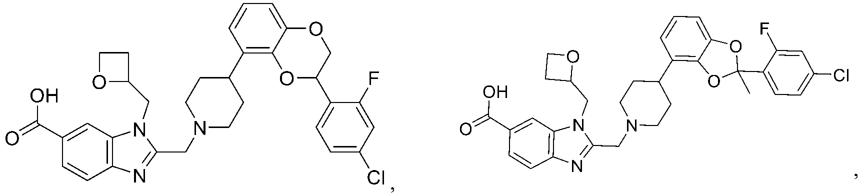

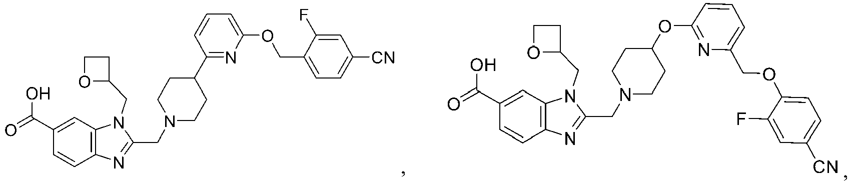

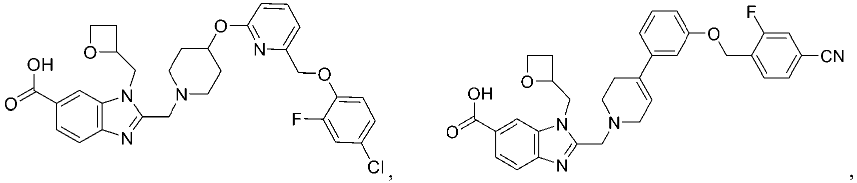

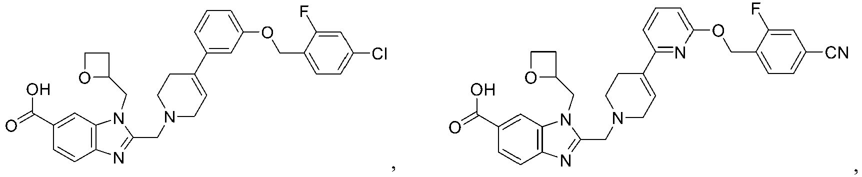

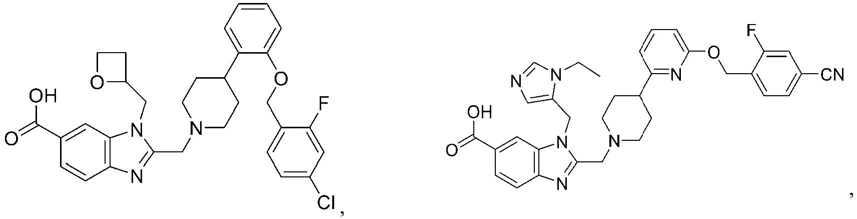

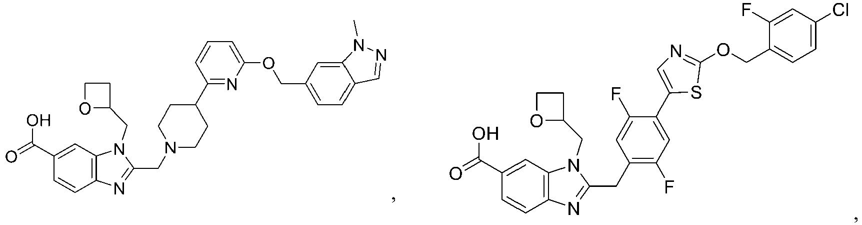

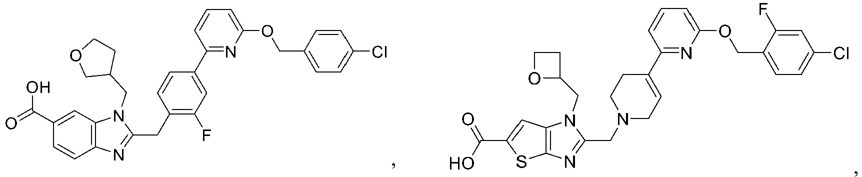

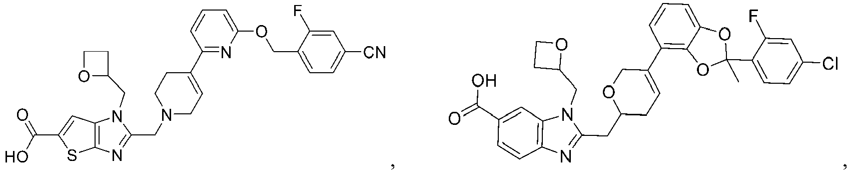

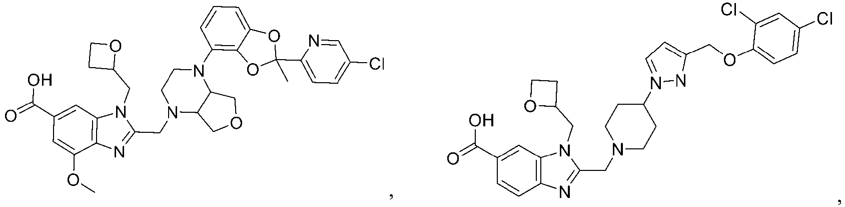

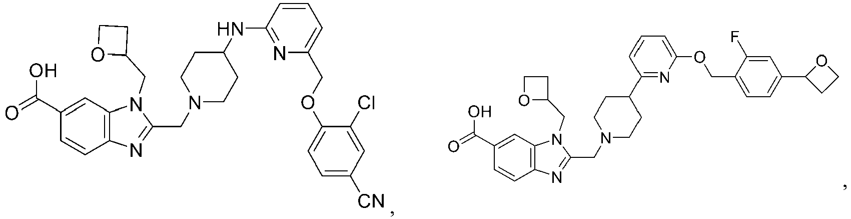

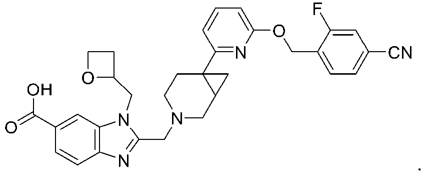

In other embodiments, the compound represented by formula I or a pharmaceutically acceptable salt thereof is selected from 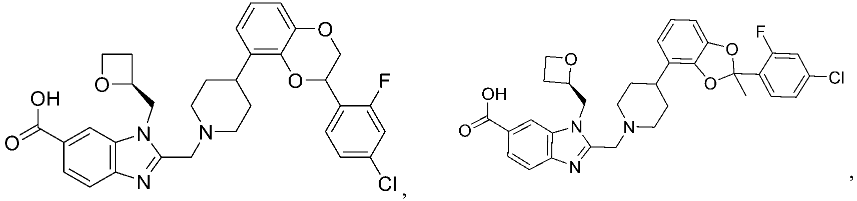 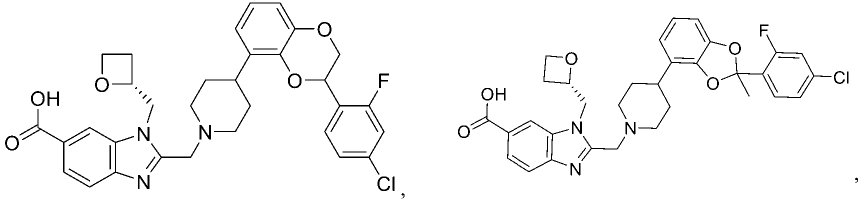 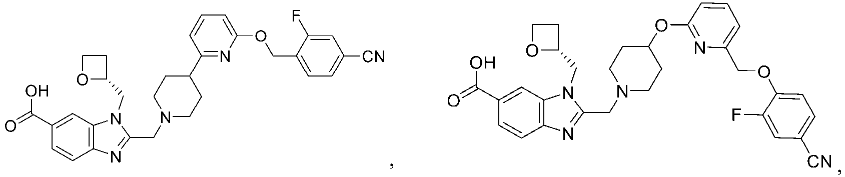 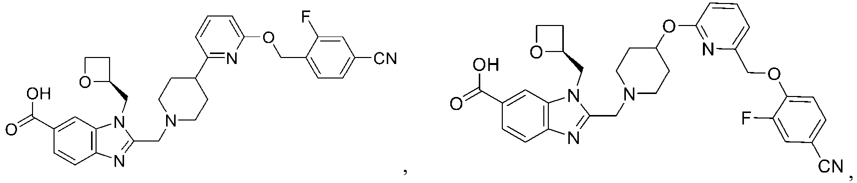 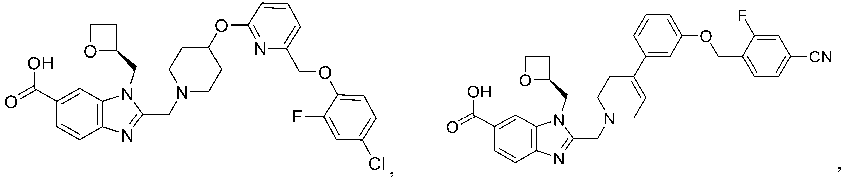 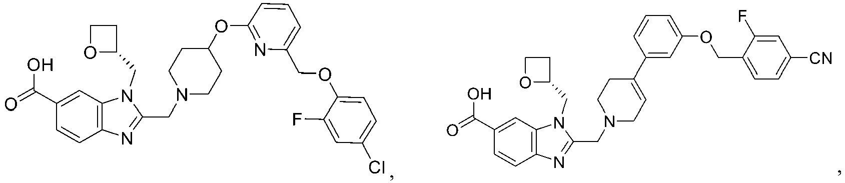 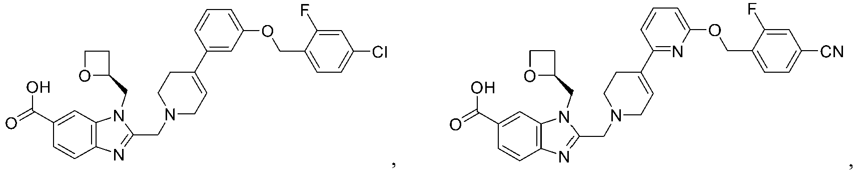 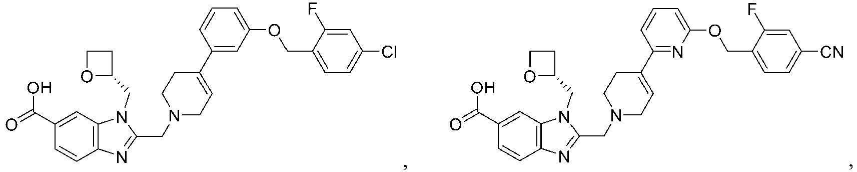 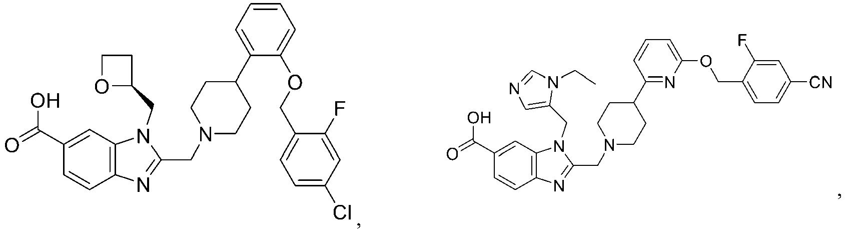 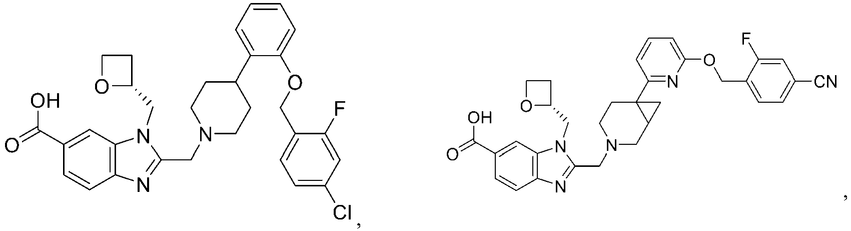 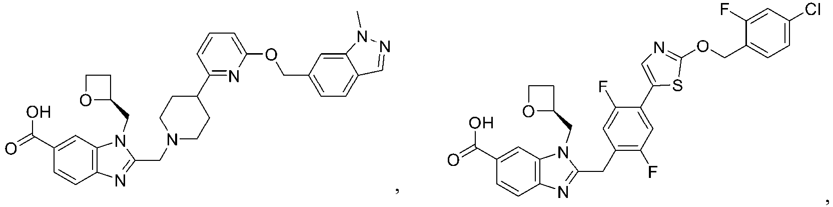 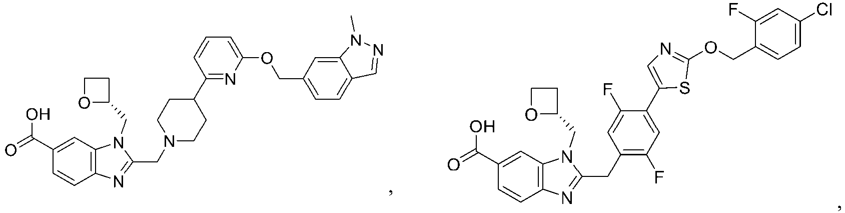 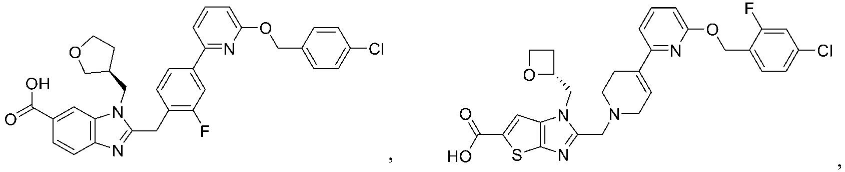 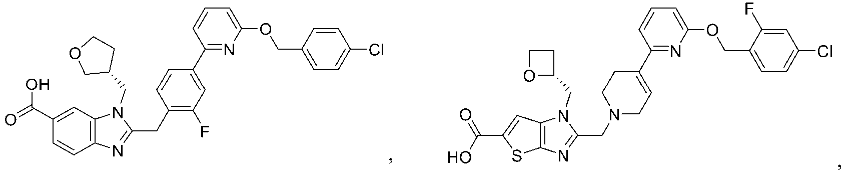 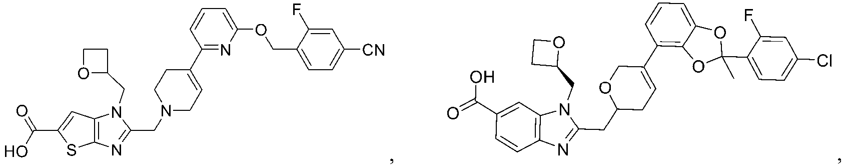 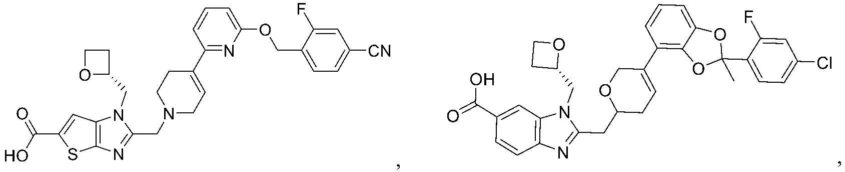 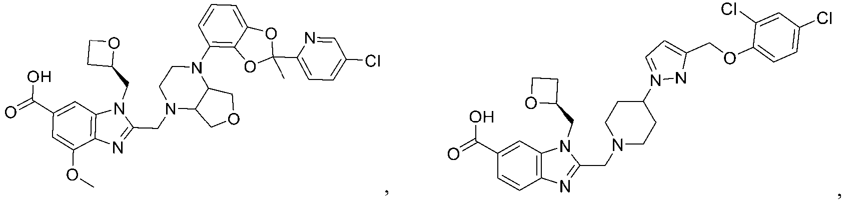 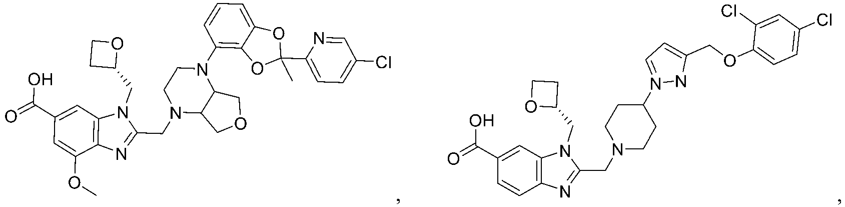 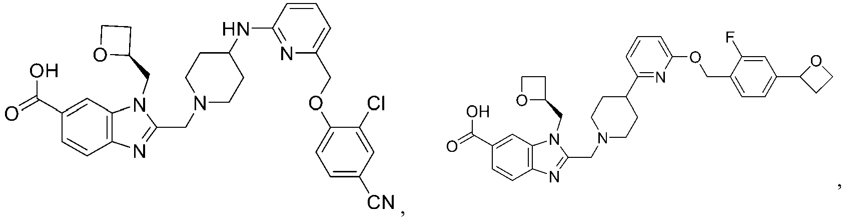 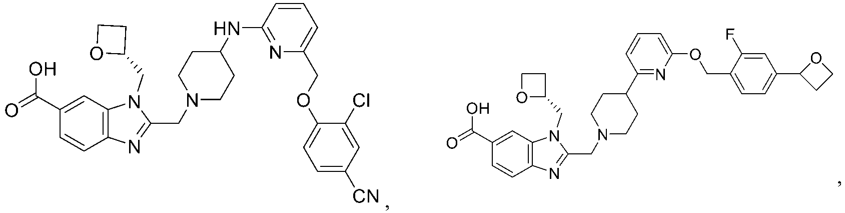 or

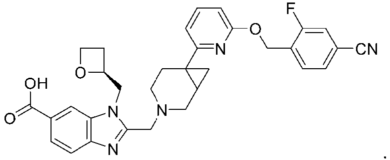

In other embodiments, the compound represented by formula I or a pharmaceutically acceptable salt thereof is selected from

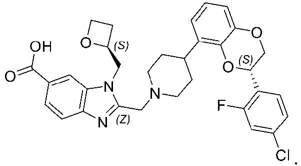

In another aspect, some embodiments provide pharmaceutical compositions comprising 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid as an active agent, and an acid-resistant compound.

Furthermore, the weight ratio of the acid-resistant compound to the active ingredient in the pharmaceutical composition described in the present disclosure is 1:1 to 1:40, and can be 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39, 1:40, or any value between any two of the foregoing values.

In some embodiments, the weight ratio of the acid-resistant compound to the active ingredient in the pharmaceutical composition is 1:1 to 1:10. In some embodiments, the weight ratio of the acid-resistant compound to the active ingredient in the pharmaceutical composition is 1:2 to 1:10. In some embodiments, the weight ratio of the acid-resistant compound to the active ingredient in the pharmaceutical composition is 1:2. In some embodiments, the weight ratio of the acid-resistant compound to the active ingredient in the pharmaceutical composition is 1:3. In some embodiments, the weight ratio of the acid-resistant compound to the active ingredient in the pharmaceutical composition is 1:4.

In some embodiments, the pharmaceutical composition comprises 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid as an active agent, and sodium carbonate.

In some embodiments, the pharmaceutical composition comprises 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid as an active agent, and sodium carbonate, wherein the weight ratio of sodium carbonate to the active ingredient is 1:1 to 1:40.

In another aspect, in some embodiments, the content of the active ingredient is 1-30%, and can be 1.0%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17%, 18%, 19%, 20%, 21%, 22%, 23%, 24%, 25%, 2%, 27%, 28%, 29%, 30%, or any value between any two of the foregoing values. In some embodiments, the active ingredient accounts for 5-15% by weight of the pharmaceutical composition. In some embodiments, the active ingredient accounts for 10-25% by weight of the pharmaceutical composition.

In some embodiments, the content of the acid-resistant compound is 0.5-10%, and can be 0.5%, 0.7%, 0.9%, 1%, 1.2%, 1.4%, 1.6%, 1.8%, 2%, 2.2%, 2.4%, 2.6%, 2.8%, 3%, 3.2%, 3.4%, 3.6%, 3.8%, 4%, 4.2%, 4.4%, 4.6%, 4.8%, 5%, 5.2%, 5.4%, 5.6%, 5.8%, 6%, 6.2%, 6.4%, 6.6%, 6.8%, 7%, 7.2%, 7.4%, 7.6%, 7.8%, 8%, 8.2%, 8.4%, 8.6%, 8.8%, 9%, 9.2%, 9.4%, 9.6%, 9.8%, 10%, or any value between any two of the foregoing values. In some embodiments, the acid-resistant compound accounts for 1-10% by weight of the pharmaceutical composition. In some embodiments, the acid-resistant compound accounts for 2-6% by weight of the pharmaceutical composition.

In some embodiments, the pharmaceutical composition comprises:
1) 1%-30% of a compound represented by formula I or a pharmaceutically acceptable salt thereof as an active ingredient;
2) 20-95% of a filler;
3) 0.5-10% of an acid-resistant compound;
4) 1-20% of a disintegrant; and
5) 0.5-10% of a binder.

In some embodiments, the pharmaceutical composition comprises:
1) 1%-30% of a compound represented by formula I or a pharmaceutically acceptable salt thereof as an active ingredient;
2) 20-95% of a filler, wherein the filler is selected from microcrystalline cellulose and mannitol;
3) 0.5-10% of an acid-resistant compound;
4) 1-20% of a disintegrant, wherein the disintegrant is selected from croscarmellose sodium; and
5) 0.5-10% of a binder, wherein the binder is selected from povidone.

In some embodiments, the pharmaceutical composition comprises:
1) 1%-30% of a compound represented by formula I or a pharmaceutically acceptable salt thereof as an active ingredient;
2) 20-95% of a filler;
3) 0.5-10% of an acid-resistant compound;
4) 1-20% of a disintegrant; and
5) 0.5-10% of a binder, wherein the weight ratio of the acid-resistant compound to the active ingredient is 1:1-1:40.

In some embodiments, the pharmaceutical composition comprises:
1) 1%-30% of a compound represented by formula I or a pharmaceutically acceptable salt thereof as an active ingredient;
2) 20-95% of a filler, wherein the filler is selected from microcrystalline cellulose and mannitol;
3) 0.5-10% of sodium carbonate as an acid-resistant compound;
4) 1-20% of a disintegrant, wherein the disintegrant is selected from croscarmellose sodium; and
5) 0.5-10% of a binder, wherein the binder is selected from povidone.

In some embodiments, the pharmaceutical composition comprises:
1) 1%-30% of a compound represented by formula I or a pharmaceutically acceptable salt thereof as an active ingredient;
2) 20-95% of a filler, wherein the filler is selected from microcrystalline cellulose and mannitol;
3) 0.5-10% of sodium carbonate as an acid-resistant compound;
4) 1-20% of a disintegrant, wherein the disintegrant is selected from croscarmellose sodium; and
5) 0.5-10% of a binder, wherein the binder is selected from povidone, and the weight ratio of the acid-resistant compound to the active ingredient is 1:1-1:40.

In some embodiments, the pharmaceutical composition comprises:
1) 1%-30% of 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid or a pharmaceutically acceptable salt thereof as an active ingredient;
2) 20-95% of a filler;
3) 0.5-10% of an acid-resistant compound;
4) 1-20% of a disintegrant; and
5) 0.5-10% of a binder.

In some embodiments, the pharmaceutical composition comprises:
1) 1%-30% of 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid or a pharmaceutically acceptable salt thereof as an active ingredient;
2) 20-95% of a filler;
3) 0.5-10% of an acid-resistant compound;
4) 1-20% of a disintegrant; and
5) 0.5-10% of a binder, wherein the weight ratio of the acid-resistant compound to the active ingredient is 1:1-1:40.

In other embodiments, one or more atoms of the aforementioned compound represented by formula I or a pharmaceutically acceptable salt thereof are replaced with isotopes having an atomic weight or mass number different from those of atoms ordinarily found in nature to form isotopically substituted derivatives. In some embodiments, such isotopically substituted derivatives are deuterated analogs.

The pharmaceutical composition of the present disclosure enables sustained and slow release of the active ingredient from the formulation (pharmaceutical composition), avoiding drastic fluctuations in plasma drug concentration and resultant erratic effective blood levels after frequent administration of conventional or immediaterelease formulations. Meanwhile, the slowly released active ingredient from the formulation (pharmaceutical composition) can effectively mitigate adverse in-vivo absorption side effects upon GLP-1R administration, including gastrointestinal adverse reactions (e.g., nausea, vomiting, diarrhea) and liver function impairment, thereby improving patient medication compliance.

In some embodiments, administration of the compound represented by formula I or a pharmaceutically acceptable salt thereof to a subject provides a Tₘₐₓ of at least 4 h, such as 4 h, 4.5 h, 5 h, 5.5 h, 6 h, 6.5 h, 7 h, 7.5 h, 8 h, 8.5 h, 9 h, 9.5 h, 10 h, 10.5 h, 11 h, 11.5 h, 12 h, or any value between any two of the foregoing values.

In some embodiments, oral administration of the compound represented by formula I or a pharmaceutically acceptable salt thereof to a population of healthy fasted subjects provides a Tₘₐₓ of at least 4 h, such as 4 h, 4.5 h, 5 h, 5.5 h, 6 h, 6.5 h, 7 h, 7.5 h, 8 h, 8.5 h, 9 h, 9.5 h, 10 h, 10.5 h, 11 h, 11.5 h, 12 h, or any value between any two of the foregoing values.

In some embodiments, oral administration of the compound represented by formula I or a pharmaceutically acceptable salt thereof to a population of healthy fasted subjects provides a Tₘₐₓ ranging from 6 h to 12 h.

In some embodiments, oral administration of the compound represented by formula I or a pharmaceutically acceptable salt thereof at a dose of 90 mg to a population of healthy fasted subjects provides a Tₘₐₓ of at least 4 h.

In some embodiments, oral administration of the compound represented by formula I or a pharmaceutically acceptable salt thereof at a dose of 90 mg to a population of healthy fasted subjects provides a Tₘₐₓ ranging from 4 h to 12, such as 4 h, 4.5 h, 5 h, 5.5 h, 6 h, 6.5 h, 7 h, 7.5 h, 8 h, 8.5 h, 9 h, 9.5 h, 10 h, 10.5 h, 11 h, 11.5 h, 12 h, or any value between any two of the foregoing values.

In some embodiments, oral administration of the compound represented by formula I or a pharmaceutically acceptable salt thereof at a dose of 90 mg to a population of healthy fasted subjects provides a Tₘₐₓ ranging from 6 h to 12 h. In some embodiments, oral administration of the compound represented by formula I or a pharmaceutically acceptable salt thereof at a dose of 90 mg to a population of healthy fasted subjects provides a Tₘₐₓ of 6 h.

In some embodiments, administration of compound A or a pharmaceutically acceptable salt thereof to a subject provides a Tₘₐₓ ranging from 4 h to 12 h.

In some embodiments, oral administration of compound A or a pharmaceutically acceptable salt thereof to a population of healthy fasted subjects provides a Tₘₐₓ ranging from 4 h to 12 h.

In some embodiments, oral administration of compound A or a pharmaceutically acceptable salt thereof at a dose of 90 mg to a population of healthy fasted subjects provides a Tₘₐₓ ranging from 4 h to 12 h.

In some embodiments, oral administration of compound A or a pharmaceutically acceptable salt thereof at a dose of 90 mg to a population of healthy fasted subjects provides a Tₘₐₓ ranging from 6 h to 12 h.

In some embodiments, oral administration of compound A or a pharmaceutically acceptable salt thereof at a dose of 90 mg to a population of healthy fasted subjects provides a Tₘₐₓ of 6 h.

In another aspect, in some embodiments, oral administration of the compound represented by formula I or a pharmaceutically acceptable salt thereof to a population of healthy fed subjects provides a Tₘₐₓ of at least 7 h, such as 8 h, 8.5 h, 9 h, 9.5 h, 10 h, 10.5 h, 11 h, 11.5 h, 12 h, 12.5 h, 13 h, 13.5 h, 14 h, 14.5 h, 15 h, 15.5 h, 16 h, or any value between any two of the foregoing values.

In some embodiments, oral administration of the compound represented by formula I or a pharmaceutically acceptable salt thereof to a population of healthy fed subjects provides a Tₘₐₓ ranging from 7 h to 16 h. In some embodiments, oral administration of the compound represented by formula I or a pharmaceutically acceptable salt thereof to a population of healthy fed subjects provides a Tₘₐₓ of 12 h.

In some embodiments, oral administration of the compound represented by formula I or a pharmaceutically acceptable salt thereof at a dose of 90 mg to a population of healthy fed subjects provides a Tₘₐₓ of at least 7 h.

In some embodiments, oral administration of the compound represented by formula I or a pharmaceutically acceptable salt thereof at a dose of 90 mg to a population of healthy fed subjects provides a Tₘₐₓ ranging from 7 h to 16 h. In some embodiments, oral administration of the compound represented by formula I or a pharmaceutically acceptable salt thereof at a dose of 90 mg to a population of healthy fed subjects provides a Tₘₐₓ of 12 h.

In some embodiments, oral administration of compound A or a pharmaceutically acceptable salt thereof at a dose of 90 mg to a population of healthy fed subjects provides a Tₘₐₓ ranging from 7 h to 16 h, such as 8 h, 8.5 h, 9 h, 9.5 h, 10 h, 10.5 h, 11 h, 11.5 h, 12 h, 12.5 h, 13 h, 13.5 h, 14 h, 14.5 h, 15 h, 15.5 h, 16 h, or any value between any two of the foregoing values.

In some embodiments, oral administration of compound A or a pharmaceutically acceptable salt thereof at a dose of 90 mg to a population of healthy fed subjects provides a Tₘₐₓ of 12 h.

The time point Tmax at which the pharmaceutical composition described in the present disclosure reaches its maximum concentration in the blood after oral administration is expressed as an arithmetic mean value. In some embodiments, the time point Tmax at which the aforementioned pharmaceutical composition reaches its maximum concentration in the blood after oral administration is expressed as a geometric mean value.

In some embodiments, oral administration of the compound represented by formula I or a pharmaceutically acceptable salt thereof at a dose of 90 mg to a population of healthy subjects provides a T_{1/2} ranging from 6 h to 8 h.

In some embodiments, oral administration of compound A or a pharmaceutically acceptable salt thereof at a dose of 90 mg to a population of healthy subjects provides a T_{1/2} ranging from 6 h to 8 h.

In some embodiments, oral administration of the compound represented by formula I or a pharmaceutically acceptable salt thereof at a dose of 90 mg to a population of healthy fasted subjects provides an average plasma Cₘₐₓ of 1370±548 ng/mL.

In some embodiments, oral administration of compound A or a pharmaceutically acceptable salt thereof at a dose of 90 mg to a population of healthy fasted subjects provides an average plasma Cₘₐₓ of 1370±548 ng/mL.

In some embodiments, oral administration of the compound represented by formula I or a pharmaceutically acceptable salt thereof at a dose of 90 mg to a population of healthy fed subjects provides an average plasma Cₘₐₓ of 810±280 ng/mL.

In some embodiments, oral administration of compound A or a pharmaceutically acceptable salt thereof at a dose of 90 mg to a population of healthy fed subjects provides an average plasma Cₘₐₓ of 810±280 ng/mL.

In some embodiments, oral administration of the compound represented by formula I or a pharmaceutically acceptable salt thereof at a dose of 90 mg to a population of healthy fasted subjects provides an average plasma AUC₀₋ₜ of 22700±3382 h*ng/mL.

In some embodiments, oral administration of compound A or a pharmaceutically acceptable salt thereof at a dose of 90 mg to a population of healthy fasted subjects provides an average plasma AUC₀₋ₜ of 22700±3382 h*ng/mL.

In some embodiments, oral administration of the compound represented by formula I or a pharmaceutically acceptable salt thereof at a dose of 90 mg to a population of healthy fed subjects provides an average plasma AUC₀₋ₜ of 17900±4439 h*ng/mL.

In some embodiments, oral administration of compound A or a pharmaceutically acceptable salt thereof at a dose of 90 mg to a population of healthy fed subjects provides an average plasma AUC₀₋ₜ of 17900±4439 h*ng/mL.

In some embodiments, oral administration of the compound represented by formula I or a pharmaceutically acceptable salt thereof at a dose of 90 mg to a population of healthy fasted subjects provides an average plasma AUC_{0-∞} of 23200±3387 h*ng/mL.

In some embodiments, oral administration of compound A or a pharmaceutically acceptable salt thereof at a dose of 90 mg to a population of healthy fasted subjects provides an average plasma AUC_{0-∞} of 23200±3387 h*ng/mL.

In some embodiments, oral administration of the compound represented by formula I or a pharmaceutically acceptable salt thereof at a dose of 90 mg to a population of healthy fed subjects provides an average plasma AUC_{0-∞} of 20500±2112 h*ng/mL.

In some embodiments, oral administration of compound A or a pharmaceutically acceptable salt thereof at a dose of 90 mg to a population of healthy fed subjects provides an average plasma AUC_{0-∞} of 20500±2112 h*ng/mL.

In another aspect, in some embodiments, oral administration of the compound represented by formula I or a pharmaceutically acceptable salt thereof at a dose of 60 mg to a population of healthy subjects provides a Tₘₐₓ of at least 7 h.

In some embodiments, oral administration of the compound represented by formula I or a pharmaceutically acceptable salt thereof at a dose of 60 mg to a population of healthy subjects provides a Tₘₐₓ ranging from 7 h to 16 h, such as 8 h, 8.5 h, 9 h, 9.5 h, 10 h, 10.5 h, 11 h, 11.5 h, 12 h, 12.5 h, 13 h, 13.5 h, 14 h, 14.5 h, 15 h, 15.5 h, 16 h, or any value between any two of the foregoing values.

In some embodiments, oral administration of the compound represented by formula I or a pharmaceutically acceptable salt thereof at a dose of 60 mg to a population of healthy subjects provides a Tₘₐₓ ranging from 9 h to 12 h, such as 9h, 9.5h, 10h, 10.5h, 11h, 11.5h, 12h, or any value between any two of the foregoing values.

In some embodiments, oral administration of the compound represented by formula I or a pharmaceutically acceptable salt thereof at a dose of 60 mg to a population of healthy subjects provides a Tₘₐₓ ranging from 10 h to 12 h.

In some embodiments, oral administration of compound A or a pharmaceutically acceptable salt thereof at a dose of 60 mg to a population of healthy subjects provides a Tₘₐₓ of at least 7 h.

The time point Tmax at which the pharmaceutical composition described in the present disclosure reaches its maximum concentration in the blood after oral administration is expressed as an arithmetic mean value. In some embodiments, the time point Tmax at which the aforementioned pharmaceutical composition reaches its maximum concentration in the blood after oral administration is expressed as a geometric mean value.

In some embodiments, oral administration of compound A or a pharmaceutically acceptable salt thereof at a dose of 60 mg to a population of healthy subjects provides a mean Tₘₐₓ ranging from 7 h to 15 h.

In some embodiments, oral administration of compound A or a pharmaceutically acceptable salt thereof at a dose of 60 mg to a population of healthy subjects provides a geometric mean Tₘₐₓ ranging from 7 h to 15 h.

In some embodiments, oral administration of the compound represented by formula I or a pharmaceutically acceptable salt thereof at a dose of 60 mg to a population of healthy subjects provides a mean plasma Cₘₐₓ of 480 ng/mL.

In some embodiments, oral administration of the compound represented by formula I or a pharmaceutically acceptable salt thereof at a dose of 90 mg to a population of healthy subjects provides a mean plasma AUC₀₋ₜ of 9437 h*ng/mL.

In some embodiments, oral administration of compound A or a pharmaceutically acceptable salt thereof at a dose of 90 mg to a population of healthy subjects provides a mean plasma AUC₀₋ₜ of 9437 h*ng/mL.

In another aspect, in some embodiments, oral administration of the compound represented by formula I or a pharmaceutically acceptable salt thereof to a population of fed subjects provides a Tₘₐₓ of at least 7 h, such as 8 h, 8.5 h, 9 h, 9.5 h, 10 h, 10.5 h, 11 h, 11.5 h, 12 h, 12.5 h, 13 h, 13.5 h, 14 h, 14.5 h, 15 h, 15.5 h, 16 h, or any value between any two of the foregoing values.

In some embodiments, oral administration of the compound represented by formula I or a pharmaceutically acceptable salt thereof to a population of subjects provides a Tₘₐₓ of at least 6 h, such as 6 h, 6.5 h, 7 h, 7.5 h, 8 h, 8.5 h, 9 h, 9.5 h, 10 h, 10.5 h, 11 h, 11.5 h, 12 h, 12.5 h, 13 h, 13.5 h, 14 h, 14.5 h, 15 h, 15.5 h, 16 h, or any value between any two of the foregoing values.

In some embodiments, the unit dose of the pharmaceutical composition is 0.001 mg to 1000 mg.

In some embodiments, the pharmaceutical composition comprises 0.01% to 99.99% of the aforementioned compound, or a pharmaceutically acceptable salt thereof, or an isotopically substituted derivative thereof, based on the total weight of the composition. In some embodiments, the pharmaceutical composition comprises 0.1% to 99.9% of the aforementioned compound, or a pharmaceutically acceptable salt thereof, or an isotopically substituted derivative thereof. In some embodiments, the pharmaceutical composition comprises 0.5% to 99.5% of the aforementioned compound, or a pharmaceutically acceptable salt thereof, or an isotopically substituted derivative thereof. In some embodiments, the pharmaceutical composition comprises 1% to 99% of the aforementioned compound, or a pharmaceutically acceptable salt thereof, or an isotopically substituted derivative thereof. In some embodiments, the pharmaceutical composition comprises 2% to 98% of the aforementioned compound, or a pharmaceutically acceptable salt thereof, or an isotopically substituted derivative thereof.

In some embodiments, the pharmaceutical composition comprises 0.01% to 99.99% of pharmaceutically acceptable excipients based on the total weight of the composition. In some embodiments, the pharmaceutical composition comprises 0.1% to 99.9% of pharmaceutically acceptable excipients. In some embodiments, the pharmaceutical composition comprises 0.5% to 99.5% of pharmaceutically acceptable excipients. In some embodiments, the pharmaceutical composition comprises 1% to 99% of pharmaceutically acceptable excipients. In some embodiments, the pharmaceutical composition comprises 2% to 98% of pharmaceutically acceptable excipients.

Another aspect of the present disclosure further provides use of the aforementioned pharmaceutical composition in the manufacture of a drug for preventing and/or treating Type I diabetes mellitus, Type II diabetes mellitus, malnutrition-related diabetes mellitus, diabetic complications, obesity, hyperglycemia, impaired glucose tolerance, cardiovascular diseases, hyperlipidemia, cerebral infarction, stroke, non-alcoholic steatohepatitis (NASH), Parkinson's disease, dementia, insulin resistance and hepatic insulin resistance. Preferentially, the use is in the manufacture of a drug for treating and/or preventing Type I diabetes mellitus, Type II diabetes mellitus, obesity, diabetic complications, non-alcoholic steatohepatitis and cardiovascular diseases.

In another aspect, the present disclosure further provides a method for preventing and/or treating diseases selected from Type I diabetes mellitus, Type II diabetes mellitus, malnutrition-related diabetes mellitus, diabetic complications, obesity, hyperglycemia, impaired glucose tolerance, cardiovascular diseases, hyperlipidemia, cerebral infarction, stroke, non-alcoholic steatohepatitis (NASH), Parkinson's disease, dementia, insulin resistance and hepatic insulin resistance, which comprises administering to a subject an effective amount of the aforementioned pharmaceutical composition.

In another aspect, the present disclosure provides the aforementioned pharmaceutical composition for use in the prevention and/or treatment of Type I diabetes mellitus, Type II diabetes mellitus, malnutrition-related diabetes mellitus, diabetic complications, obesity, hyperglycemia, impaired glucose tolerance, cardiovascular diseases, hyperlipidemia, cerebral infarction, stroke, non-alcoholic steatohepatitis (NASH), Parkinson's disease, dementia, insulin resistance and hepatic insulin resistance.

In another aspect, the present disclosure provides a sustained-release pharmaceutical composition comprising a compound represented by formula I or a pharmaceutically acceptable salt thereof as an active ingredient, 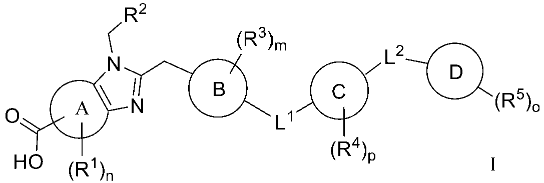 wherein,
ring A is selected from phenyl or 5- or 6-membered heteroaryl;
each R¹ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
R² is selected from 3- to 6-membered heterocycloalkyl or 5- or 6-membered heteroaryl, wherein the heterocycloalkyl or heteroaryl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
ring B is selected from 3- to 6-membered heterocycloalkyl, phenyl, or 5- or 6-membered heteroaryl;
each R³ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl,
or any two R³ groups, together with the adjacent atoms to which they are attached, form C₃₋₆ cycloalkyl or 3- to 6-membered heterocycloalkyl, wherein the cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
L¹ is selected from -O-, -S-, -C(O)O-, -OC(O)-, -C(O)-, -S(O)-, -S(O)₂-, -C(R^{1a}R^{1b})-, -OC(R^{1a}R^{1b})-, -C(R^{1a}R^{1b})O-, -C(O)N(R^{1c})-, -N(R^{1c})(O)C-, -S(O)₂N(R^{1d})-, -N(R^{1e})- or a bond,
each of R^{1a}, R^{1b}, R^{1c}, R^{1d} and R^{1e} is independently selected from hydrogen, halogen, hydroxy, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl or C₁₋₆ alkoxy;
ring C is selected from C₆₋₁₀ aryl or 5- or 10-membered heteroaryl;
each R⁴ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
L² is selected from-O-, -S-, -C(O)O-, -OC(O)-, -C(O)-, -S(O)-, -S(O)₂-, -C(R^{2a}R^{2b})-, -OC(R^{2a}R^{2b})-, -C(R^{2a}R^{2b})O-, -C(O)N(R^{2c})-, -N(R^{2c})(O)C-, -S(O)₂N(R^{2d})-, -N(R^{2e})- or a bond;
each of R^{2a}, R^{2b}, R^{2c}, R^{2d} and R^{2e} is independently selected from hydrogen, halogen, hydroxy, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl or C₁₋₆ alkoxy;
ring D is selected from C₆₋₁₀ aryl or 5- or 10-membered heteroaryl;
each R⁵ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
n is selected from 0, 1, 2, 3 or 4;
m is selected from 0, 1, 2, 3 or 4;
o is selected from 0, 1, 2, 3 or 4;
p is selected from 0, 1, 2, 3 or 4.

In some embodiments, the pharmaceutical composition comprises 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid as an active agent.

The time point Tmax at which the sustained-release pharmaceutical composition of the present disclosure reaches its maximum concentration in the blood after oral administration is expressed as an arithmetic mean value. In some embodiments, the time point Tmax at which the aforementioned pharmaceutical composition reaches its maximum concentration in the blood after oral administration is expressed as a geometric mean value.

In some embodiments, upon oral administration of the compound represented by formula I or a pharmaceutically acceptable salt thereof to a subject, the sustained-release pharmaceutical composition provides a Tₘₐₓ of at least 4 h, such as 4 h, 4.5 h, 5 h, 5.5 h, 6 h, 6.5 h, 7 h, 7.5 h, 8 h, 8.5 h, 9 h, 9.5 h, 10 h, 10.5 h, 11 h, 11.5 h, 12 h, or any value between any two of the foregoing values.

In some embodiments, upon oral administration of the compound represented by formula I or a pharmaceutically acceptable salt thereof to a population of fasted subjects, the sustained-release pharmaceutical composition provides a Tₘₐₓ of at least 4 h, for example, 4 h, 4.5 h, 5 h, 5.5 h, 6 h, 6.5 h, 7 h, 7.5 h, 8 h, 8.5 h, 9 h, 9.5 h, 10 h, 10.5 h, 11 h, 11.5 h, 12 h, or any value between any two of the foregoing values.

In some embodiments, upon oral administration of the compound represented by formula I or a pharmaceutically acceptable salt thereof at a dose of 90 mg to a population of healthy fasted subjects, the sustained-release pharmaceutical composition provides a Tₘₐₓ of at least 4 h.

In some embodiments, upon oral administration of the compound represented by formula I or a pharmaceutically acceptable salt thereof at a dose of 90 mg to a population of healthy fasted subjects, the sustained-release pharmaceutical composition provides a Tₘₐₓ ranging from 4 h to 12h, for example, 4 h, 4.5 h, 5 h, 5.5 h, 6 h, 6.5 h, 7 h, 7.5 h, 8 h, 8.5 h, 9 h, 9.5 h, 10 h, 10.5 h, 11 h, 11.5 h, 12 h, or any value between any two of the foregoing values.

In some embodiments, upon oral administration of the compound represented by formula I or a pharmaceutically acceptable salt thereof at a dose of 90 mg to a population of healthy fasted subjects, the sustained-release pharmaceutical composition provides a Tₘₐₓ ranging from 6 h to 12 h.

In some embodiments, upon oral administration of the compound represented by formula I or a pharmaceutically acceptable salt thereof at a dose of 90 mg to a population of healthy fasted subjects, the sustained-release pharmaceutical composition provides a median Tₘₐₓ of 6 h.

In some embodiments, upon oral administration of compound A or a pharmaceutically acceptable salt thereof at a dose of 90 mg to a population of healthy fasted subjects, the sustained-release pharmaceutical composition provides a Tₘₐₓ ranging from 4 h to 12 h.

In some embodiments, upon oral administration of compound A or a pharmaceutically acceptable salt thereof at a dose of 90 mg to a population of healthy fasted subjects, the sustained-release pharmaceutical composition provides a Tₘₐₓ ranging from 6 h to 12 h.

In some embodiments, upon oral administration of compound A or a pharmaceutically acceptable salt thereof at a dose of 90 mg to a population of healthy fasted subjects, the sustained-release pharmaceutical composition provides a Tₘₐₓ of 6 h.

In some embodiments, upon oral administration of the compound represented by formula I or a pharmaceutically acceptable salt thereof to a population of fed subjects, the sustained-release pharmaceutical composition provides a Tₘₐₓ of at least 7 h, for example, 7h, 7.5h, 8h, 8.5h, 9h, 9.5h, 10h, 10.5h, 11h, 11.5h, 12h, 12.5h, 13h, 13.5h, 14h, 14.5h, 15h, 15.5h, 16h, or any value between any two of the foregoing values.

In some embodiments, upon oral administration of the compound represented by formula I or a pharmaceutically acceptable salt thereof at a dose of 90 mg to a population of healthy fed subjects, the sustained-release pharmaceutical composition provides a Tₘₐₓ of at least 7 h.

In some embodiments, upon oral administration of the compound represented by formula I or a pharmaceutically acceptable salt thereof at a dose of 90 mg to a population of healthy fed subjects, the sustained-release pharmaceutical composition provides a Tₘₐₓ ranging from 7 h to 16 h, such as 7 h, 7.5 h, 8 h, 8.5 h, 9 h, 9.5 h, 10 h, 10.5 h, 11 h, 11.5 h, 12 h, 12.5 h, 13 h, 13.5 h, 14 h, 14.5 h, 15 h, 15.5 h, 16 h, or any value between any two of the foregoing values.

In some embodiments, upon oral administration of the compound represented by formula I or a pharmaceutically acceptable salt thereof at a dose of 90 mg to a population of healthy fed subjects, the sustained-release pharmaceutical composition provides a Tₘₐₓ of at least 12 h.

In some embodiments, upon oral administration of compound A or a pharmaceutically acceptable salt thereof at a dose of 90 mg to a population of healthy fed subjects, the sustained-release pharmaceutical composition provides a Tₘₐₓ ranging from 7 h to 16 h.

In some embodiments, upon oral administration of compound A or a pharmaceutically acceptable salt thereof at a dose of 90 mg to a population of healthy fed subjects, the sustained-release pharmaceutical composition provides a Tₘₐₓ of 12 h.

In another aspect, in some embodiments, upon oral administration of the compound represented by formula I or a pharmaceutically acceptable salt thereof at a dose of 60 mg to a population of healthy subjects, the sustained-release pharmaceutical composition provides a Tₘₐₓ of at least 7 h.

In some embodiments, upon oral administration of the compound represented by formula I or a pharmaceutically acceptable salt thereof at a dose of 60 mg to a population of healthy subjects, the sustained-release pharmaceutical composition provides a Tₘₐₓ ranging from 7 h to 16 h, such as 7 h, 7.5 h, 8 h, 8.5 h, 9 h, 9.5 h, 10 h, 10.5 h, 11 h, 11.5 h, 12 h, 12.5 h, 13 h, 13.5 h, 14 h, 14.5 h, 15 h, 15.5 h, 16 h, or any value between any two of the foregoing values.

In some embodiments, upon oral administration of the compound represented by formula I or a pharmaceutically acceptable salt thereof at a dose of 60 mg to a population of healthy subjects, the sustained-release pharmaceutical composition provides a Tₘₐₓ of ranging from 9 h to 12 h.

In some embodiments, upon oral administration of the compound represented by formula I or a pharmaceutically acceptable salt thereof at a dose of 60 mg to a population of healthy subjects, the sustained-release pharmaceutical composition provides a Tₘₐₓ of ranging from 10 h to 12 h. In some embodiments, upon oral administration of compound A or a pharmaceutically acceptable salt thereof at a dose of 60 mg to a population of healthy subjects, the sustained-release pharmaceutical composition provides a Tₘₐₓ of at least 7 h.

In some embodiments, upon oral administration of the compound represented by formula I or a pharmaceutically acceptable salt thereof at a dose of 60 mg to a population of healthy subjects, the sustained-release pharmaceutical composition provides an average plasma Cₘₐₓ of 480 ng/mL.

In some embodiments, upon oral administration of the compound represented by formula I or a pharmaceutically acceptable salt thereof at a dose of 90 mg to a population of healthy subjects, the sustained-release pharmaceutical composition provides an average plasma AUC₀₋ₜ of 9437 h*ng/mL.

In some embodiments, upon oral administration of compound A or a pharmaceutically acceptable salt thereof at a dose of 90 mg to a population of healthy subjects, the sustained-release pharmaceutical composition provides an average plasma AUC₀₋ₜ of 9437 h*ng/mL.

In some embodiments, the sustained-release pharmaceutical composition comprises an acid-resistant compound. In some embodiments, the acid-resistant compound is selected from water-soluble acid-resistant compounds, such as sodium carbonate. In some embodiments, the sustained-release pharmaceutical composition comprises 0.5% to 10% of the acid-resistant compound. In some embodiments, the weight ratio of the acid-resistant compound to the active ingredient in the sustained-release pharmaceutical composition is 1:1 to 1:40.

The "sustained-release pharmaceutical composition" as used in the present disclosure is well known to those skilled in the art; such compositions release the active ingredient slowly at a non-constant rate in a specified release medium and require half or fewer administrations compared with corresponding conventional formulations (pharmaceutical compositions).

If required, the resulting granules or uncoated core tablets may be further coated or filled into capsules and the like.

The expressions "by weight of the pharmaceutical composition" or "weight of the pharmaceutical composition" as used in the present disclosure mean that the content ranges of the active ingredient or other pharmaceutically acceptable excipients are calculated based on the weight of the tablet core excluding any coating material. The pharmaceutically acceptable salts of the compounds described in the present disclosure may be selected from inorganic or organic salts, such as tromethamine salts.

The compounds disclosed herein may exist in specific geometric or stereoisomer forms. The present disclosure contemplates all such isomers, including cis and trans isomers, (-)- and (+)-enantiomers, (R)- and (S)-enantiomers, diastereomers, D-isomers and L-isomers, as well as racemic mixtures and other mixtures such as enantiomer-enriched or diastereomer-enriched mixtures, all of which fall within the scope of the present disclosure. Additional asymmetric carbon atoms may be present in substituents such as alkyl. All such isomers and mixtures thereof are encompassed within the scope of the present disclosure. The compounds of the present disclosure containing asymmetric carbon atoms may be isolated in optically pure form or racemic form. Optically pure forms can be resolved from racemic mixtures or synthesized using chiral starting materials or chiral reagents.

Optically active (R)- and (S)-isomers as well as D and L isomers can be prepared via chiral synthesis, chiral resolving agents or other conventional techniques. Where a single enantiomer of a compound herein is desired, it may be prepared by asymmetric synthesis or derivatization with a chiral auxiliary, followed by separation of the resulting diastereomeric mixture and cleavage of the auxiliary group to afford the desired pure enantiomer. Alternatively, where the molecule bears a basic functional group (e.g., amino group) or an acidic functional group (e.g., carboxyl group), diastereomeric salts can be formed with an appropriate optically active acid or base; the diastereomers are subsequently resolved by conventional methods well known in the art to recover the pure target enantiomer. In addition, separation of enantiomers and diastereomers is commonly achieved by chromatography employing a chiral stationary phase, optionally combined with chemical derivatization (e.g., carbamate formation from amines).

In the chemical structure of the compounds described in the present disclosure, the bond " 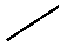 " indicates that the configuration is not specified. That is, if there are chiral isomers in the chemical structure, the bond " 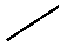 " can be " 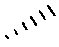 " or " 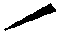 ", or can contain both configurations of " 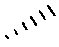 " and " 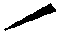 ".

For example, 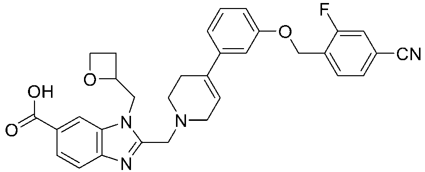 has chiral isomers, comprising two configurations " 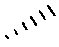 " and " 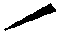 ", such as 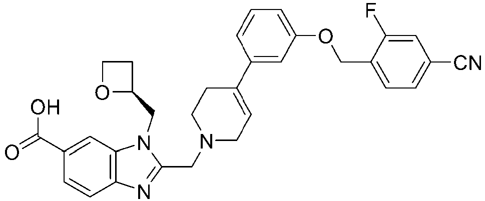 and

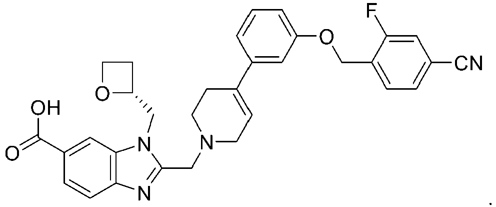

In another aspect, where a compound comprises more than one chiral carbon center, multiple diastereomers will exist. By way of example, 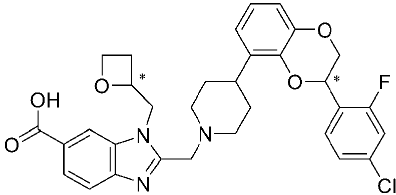 the compound comprises two chiral carbon centers and encompasses numerous diastereomers such as 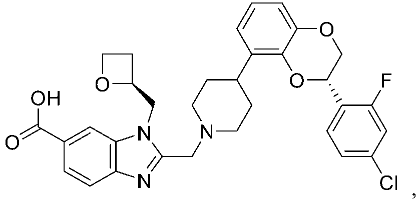 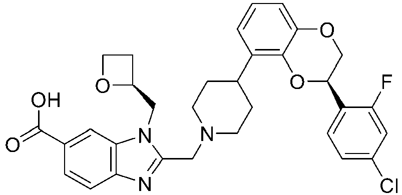 and the like.

The compounds and intermediates of the present disclosure may also exist in various tautomeric forms, and all such forms are included within the scope of the present disclosure. The term "tautomer" or "tautomeric form" refers to structural isomers of differing energies that are interconvertible via a low energy barrier.

The present disclosure also encompasses isotopically labeled compounds identical to those described herein, wherein one or more atoms are replaced by atoms having an atomic mass or mass number different from the atomic mass or mass number ordinarily found in nature. Examples of isotopes that can be incorporated into the disclosed compounds include isotopes of hydrogen, carbon, nitrogen, oxygen, phosphorus, sulfur, fluorine, iodine and chlorine, such as ²H, ³H, ¹¹C, ¹³ C, ¹⁴ C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl, respectively.

Unless otherwise specified, when a position is specifically designated as deuterium (D), such position is understood to bear deuterium at an abundance at least 1000 times higher than the natural abundance of deuterium (0.015%) (i.e., at least 10% deuterium incorporation). In exemplary compounds, deuterium abundance may be at least 1000-fold, at least 2000-fold, at least 3000-fold, at least 4000-fold, at least 5000-fold, at least 6000-fold or even higher relative to natural deuterium abundance. The present disclosure further covers compounds of formula (I) in various deuterated forms. Each available hydrogen atom bound to a carbon atom may be independently replaced with a deuterium atom. Those skilled in the art can synthesize the deuterated form of compounds of formula (I) by referring to relevant literature. Commercially available deuterated starting materials can be used to prepare deuterated compounds of formula (I); alternatively, such compounds may be synthesized via conventional techniques using deuterated reagents including, but not limited to, deuterated borane, trideuterated borane tetrahydrofuran solution, deuterated lithium aluminum hydride, deuterated ethyl iodide and deuterated methyl iodide.

### Terms:

As used herein, the term "by weight of the pharmaceutical composition" means that the quantitative ranges for the active ingredient or other pharmaceutically acceptable excipients are calculated based on the weight of the tablet core excluding coating materials.

"Optionally" or "optional" means that the subsequently described event or circumstance may, but not necessarily, occur, and that the description includes instances where the event or circumstance occurs and does not occur. For example, "C₁₋₆ alkyl optionally substituted with halogen or cyano" indicates that halogen or cyano may be present or absent, covering both alkyl groups substituted with halogen or cyano and alkyl groups not substituted with halogen and cyano.

The term "pharmaceutically acceptable excipient" includes, but is not limited to, any adjuvant, carrier, glidant, sweetener, diluent, preservative, dye/colorant, flavoring agent, surfactant, wetting agent, dispersant, suspending agent, stabilizer, isotonic agent, solvent or emulsifier approved by the U.S. Food and Drug Administration (FDA) for human or veterinary use.

As used herein, "effective amount" or "therapeutically effective amount" refers to an amount sufficient to ameliorate or prevent the symptoms or underlying pathology of a medical disorder. An effective amount also refers to an amount sufficient to allow or facilitate a diagnosis. The effective amount for a specific human or veterinary subject varies depending on factors including the disorder to be treated, the subject's overall physical condition, administration route and dosage regimen, and the severity of adverse effects. An effective amount can be the maximum dose or dosing regimen that avoids significant side effects or toxicity.

The term "alkyl" refers to a saturated aliphatic hydrocarbon radical encompassing straight and branched-chain groups containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl and various branched isomers thereof. An alkyl group may be substituted or unsubstituted. Where substituted, substitution may occur at any available attachment site, and the substituent is preferably one or more groups independently selected from halogen, hydroxy, cyano, nitro, amino, C₁₋₆ alkyl, C₁₋₆ alkoxy, C₃₋₆ cycloalkyl or 3- to 6-membered heterocycloalkyl.

The term "cycloalkyl" refers to a saturated or partially unsaturated monocyclic or polycyclic cyclic hydrocarbon substituent having a ring containing 3 to 6 carbon atoms, such as 4 or 5 carbon atoms. Non-limiting examples of monocyclic cycloalkyl include cyclopropyl, cyclobutyl, cyclopentyl, cyclopentenyl, cyclohexyl, cyclohexenyl, cyclohexadienyl and the like; polycyclic cycloalkyl includes spiro, fused and bridged cycloalkyl groups. A cycloalkyl group may be substituted or unsubstituted. Where substituted, the substituent(s) are preferably one or more groups independently selected from halogen, hydroxy, cyano, nitro, amino, C₁₋₆alkyl, C₁₋₆ alkoxy, C₃₋₆ cycloalkyl or 3- to 6-membered heterocycloalkyl.

The term "heterocycloalkyl" refers to a saturated or partially unsaturated monocyclic or polycyclic cyclic hydrocarbon substituent containing 3 to 6 ring atoms, for instance 4 or 5 ring atoms, wherein one or more ring atoms are heteroatoms independently selected from nitrogen, oxygen or S(O)ₘ (m is an integer of 0 to 2), excluding ring moieties containing -O-O-, -O-S- or -S-S-, with the remaining ring atoms being carbon. Non-limiting examples of "heterocycloalkyl" include: 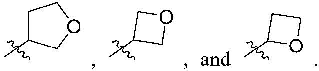 A heterocycloalkyl group may be optionally substituted or unsubstituted. Where substituted, the substituent(s) are preferably one or more groups independently selected from halogen, hydroxy, cyano, nitro, amino, C₁₋₆ alkyl, C₁₋₆ alkoxy, C₃₋₆ cycloalkyl or 3- to 6-membered heterocycloalkyl.

The term "alkoxy" denotes the group -O-(alkyl), where alkyl is defined as set forth above. Non-limiting examples include methoxy, ethoxy, propoxy and butoxy. An alkoxy group may be optionally substituted or unsubstituted. Where substituted, the substituent(s) are preferably one or more groups independently selected from halogen, hydroxy, cyano, nitro, amino, C₁₋₆ alkyl, C₁₋₆ alkoxy, C₃₋₆ cycloalkyl or 3- to 6-membered heterocycloalkyl.

The term "aryl" refers to a fully carbocyclic monocyclic or fused polycyclic (i.e., rings sharing adjacent pairs of carbon atoms) group having a conjugated π-electron system with 6 to 10 ring carbon atoms, such as phenyl and naphthyl. The aryl ring may be fused to a heteroaryl, heterocycloalkyl or cycloalkyl ring, wherein the ring attached to the parent structure is the aryl ring. Non-limiting examples include:

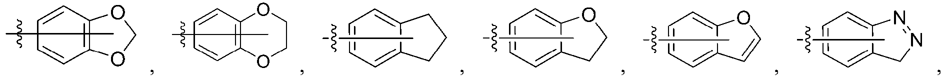

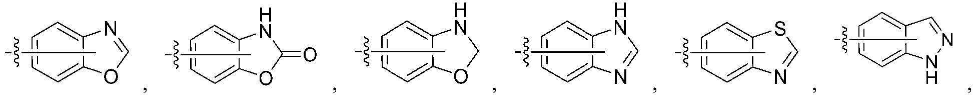

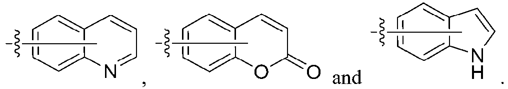

An aryl group may be substituted or unsubstituted. Where substituted, the substituent(s) are preferably one or more groups independently selected from halogen, hydroxy, cyano, nitro, amino, C₁₋₆ alkyl, C₁₋₆ alkoxy, C₃₋₆ cycloalkyl or 3 - to 6-membered heterocycloalkyl.

The term "heteroaryl" denotes a heteroaromatic system containing 5 to 10 ring atoms including 1 to 4 heteroatoms selected from oxygen, sulfur and nitrogen. Heteroaryl is preferably 5- to 10-membered, such as 7-, 8- or 9-membered, more preferably 5-membered or 6-membered. For example, non-limiting examples include:

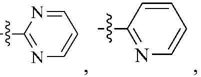

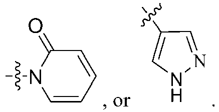

The heteroaryl ring may be fused to an aryl, heterocycloalkyl or cycloalkyl ring, with the ring bonded to the parent structure being the heteroaryl ring. Non-limiting examples include:

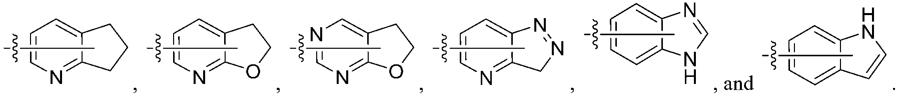

A heteroaryl group may be substituted or unsubstituted. Where substituted, the substituent(s) are preferably one or more groups independently selected from halogen, hydroxy, cyano, nitro, amino, C₁₋₆ alkyl, C₁₋₆ alkoxy, C₃₋₆ cycloalkyl or 3 - to 6-membered heterocycloalkyl.

The term "heterocycle" refers to a ring containing atoms other than carbon as ring-forming members, encompassing heterocycloalkyl and heteroaryl. "Heterocycloalkyl" and "heteroaryl" are as defined above.

The term "hydroxy" refers to the -OH group.

The term "halogen" refers to fluorine, chlorine, bromine, or iodine.

The term "cyano" refers to -CN.

The term "amino" refers to -NH₂.

The term "nitro" refers to -NO₂.

The term "oxo" refers to "=O".

"Substituted" means that one or more hydrogen atoms, preferably up to 5, more preferably 1 to 3 hydrogen atoms in a group are independently of each other replaced by a corresponding number of substituents. It is understood that the substituents are only in their possible chemical positions and those skilled in the art will be able to determine (either experimentally or theoretically) possible or impossible substitutions without undue effort.

As used herein, the singular forms "a", "an" and "the" include plural referents, and vice versa, unless the context clearly dictates otherwise.

Numerical values recited in the present disclosure are instrument-measured data and subject to inherent measurement error. In general, deviations within ±10% fall within reasonable error tolerance. Context pertaining to each numerical parameter shall be taken into consideration by way of example: for particle size of active ingredient, permissible measurement variation is no greater than ±10%, such as ±9%, ±8%, ±7%, ±6%, ±5%, ±4%, ±3%, ±2% or ±1%, with ±5% being preferred.

### Pharmacokinetic parameters

| **Parameter** | **Definition** |
|---|---|
| Cₘₐₓ | Peak concentration. Directly derived from observed plasma drug concentration-time data. |
| AUC₀₋ₜ | Area under the concentration time curve from time zero to the last quantifiable plasma drug concentration. Calculated by the linear trapezoidal rule: AUC_{(i, i+1)}= (Tᵢ₊₁-Tᵢ)(Cᵢ+Cᵢ₊₁)/2, where AUC₀₋ₜ is the sum of all AUC_{(i, i+1)}. |
| AUC_{0-∞} | Area under the concentration-time curve extrapolated from time zero to infinity. AUC_{0-∞}= AUC₀₋ₜ+ Cₜ/λ_{z} (Cₜ is the last measurable plasma drug concentration). |
| Tₘₐₓ | Time to reach maximum plasma drug concentration. Directly derived from observed plasma drug concentration time data. |
| t_{1/2z} | Terminal elimination half-life. t_{1/2z}=ln2/λ_{z}. |

All pharmaceutical excipients and reagents described herein are commercially available; for instance, hydroxypropyl methylcellulose can be purchased from commercial suppliers. Compound A: 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid is prepared in accordance with the procedures disclosed in WO2022007979, the content of which is incorporated herein by reference.

### DETAILED DESCRIPTION OF PREFERRED EMBODIMENTS

The following constitutes specific embodiments of the present disclosure. The examples are provided to further illustrate rather than limit the present disclosure, and all technical solutions equivalent thereto shall fall within the scope of protection of the present disclosure.

### Example 1

In accordance with the doses listed in Table 1, 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid (compound A), microcrystalline cellulose, mannitol, povidone, anhydrous sodium carbonate and magnesium stearate were blended, followed by dry granulation and sizing. The sized granules were then homogeneously blended with croscarmellose sodium and magnesium stearate. The resulting final blended granules were compressed into tablets.

**Table 1**

| Ingredient | Formula 1 | | Formula 2 | |
|---|---|---|---|---|
| | mg/tablet | Percentage | mg/tablet | Percentage |
| Compound A | 60 | 10.6% | 60 | 10.6% |
| Microcrystalline cellulose 112 | 217.6 | 38.3% | 228.8 | 40.3% |
| Mannitol 200SD | 217.6 | 38.3% | 228.8 | 40.3% |
| Povidone K30 | 11.2 | 2.0% | 11.2 | 2.0% |
| Anhydrous sodium carbonate | 22.4 | 3.9% | 0 | 0.0% |
| Magnesium stearate | 4.4 | 0.8% | 4.4 | 0.8% |
| Croscarmellose sodium (additionally added) | 29.5 | 5.2% | 29.5 | 5.2% |
| Magnesium stearate (additionally added) | 5.3 | 0.9% | 5.3 | 0.9% |
| Total | 568 | 100.0% | 568 | 100.0% |

### Dissolution test

Dissolution tests of tablets from formula 1 and formula 2 were performed in accordance with the Second Method (Paddle Method) for dissolution measurement specified in Appendix of Volume II of Chinese Pharmacopoeia 2020 Edition. A total of 1000 ml of pH 5.0 acetate buffer containing 0.2% cetyltrimethylammonium bromide (CTAB) was adopted as dissolution medium, and the test was conducted at a paddle rotation speed of 50 rpm under 37±0.5 °C.

**Table 2**

| Time (h) | Formula 1 | Formula 2 |
|---|---|---|
| | Dissolution (%) | |
| 0.5 | 26 | 89 |
| 0.75 | / | 89 |
| 1 | 32 | / |
| 2 | 41 | / |
| 3 | 46 | / |
| 4 | 49 | / |
| 5 | 52 | / |
| 6 | 56 | / |
| 8 | 63 | / |
| 12 | 74 | / |

| | | |
|---|---|---|
| Note: "/" indicates no sample tested | | |

The results show that compound A in formula 1 was released slowly, with a cumulative dissolution of 74% after 12 h, while formula 2 reached a dissolution of 89% after 0.75 h (45 min), reaching near-complete dissolution. Compared with formula 2, formula 1 supplemented with sodium carbonate displayed typical sustained-release properties.

### Example 2

In accordance with the doses listed in Table 3, 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid (compound A), anhydrous dibasic calcium phosphate, mannitol and povidone were blended. The mixture was subjected to wet granulation using an aqueous sodium carbonate solution followed by sizing. The sized granules were then uniformly blended with microcrystalline cellulose, croscarmellose sodium and magnesium stearate. The resulting final blended granules were compressed into tablets.

**Table 3**

| Component | Formula 3 | |
|---|---|---|
| | mg/tablet | Percentage |
| Compound A | 15 | 25 |
| Anhydrous calcium hydrogen phosphate | 14.7 | 24.5 |
| Mannitol | 14.7 | 24.5 |
| Povidone | 1.2 | 2 |
| Anhydrous sodium carbonate | 2.4 | 4 |
| Microcrystalline cellulose | 9 | 15 |
| Croscarmellose sodium (additionally added) | 2.4 | 4 |
| Magnesium stearate (additionally added) | 0.6 | 1 |
| Total | 60 | 100 |

Dissolution test was carried out in accordance with Method 1 (Basket Method) specified in the Appendix of Volume II, Chinese Pharmacopoeia 2020 Edition. A total of 900 ml of pH 5.0 acetate buffer containing 0.2% cetyltrimethylammonium bromide (CTAB) was adopted as dissolution medium, and the test was conducted at 100 rpm under 37±0.5 °C.

**Table 4**

| Time (h) | Dissolution (%) |
|---|---|
| 0.5 | 30 |
| 1 | 39 |
| 2 | 54 |
| 3 | 63 |
| 4 | 72 |
| 5 | 81 |
| 6 | 88 |
| 8 | 100 |

The results show that formula 3 had a slow release, with 100% cumulative dissolution after 8 h. Compared with formula 2, formula 1 and formula 3, which contain sodium carbonate, exhibited sustained-release properties.

### Test Example 1: Pharmacokinetic study in healthy human subjects

### 1.1 Study objective

To investigate the pharmacokinetic profiles of tablets of formula 3 following a single oral dose of 90 mg under fasted and fed conditions in healthy subjects

### 1.2 Study design

A single-center, double-blind trial design was adopted. A total of 10 healthy subjects were planned for enrollment to receive drug administration sequentially under fed and fasted states with a washout period of at least 3 days. Blood samples were collected at scheduled time points, and safety assessments were performed. Plasma drug concentrations at various post-dose time points were quantified via HPLC-MS/MS. Pharmacokinetic parameters were calculated and subjected to statistical analysis.

### 1.3 Dosing regimen

Subjects were admitted to the study site on D-2. After standardized dinner on D-2, subjects fasted overnight for 10 h with free access to drinking water, and 5-point fingertip blood glucose testing was conducted on the following day prior to standardized meals. Following standardized dinner on D-1, subjects fasted for a minimum of 10 h overnight with unrestricted drinking water. On the morning of D1, after collection of pre-dose fasting blood samples, subjects started a high-fat high-calorie meal 30 ± 1 min prior to drug administration and finished the meal within 30 min. Subjects then received formula 3 tablets or matching placebo with 240 mL of water. No water intake was permitted from 1 h pre-dose to 1 h post-dose, and all food was prohibited within 4 h post-dose. Rescue food supplementation was allowed as needed for subjects experiencing hypoglycemia.

Upon completion of the fed-phase dosing period, subjects underwent a washout period of at least 1 day prior to baseline screening for the fasting phase (scheduled on D3 after fed dosing). Subjects proceeded directly to fasting administration if no unresolved adverse events were identified. In cases of newly emergent adverse events or persistent unresolved adverse events, follow-up was continued until the investigator confirmed eligibility for fasting dosing or the subject was withdrawn from the trial. On the subsequent day, 5-point fingertip blood glucose measurement was performed; subjects received standardized dinner followed by overnight fasting of at least 10 h with free access to water. On the next morning after collection of fasting baseline blood samples, subjects were administered formula 3 tablets or placebo with 240 mL of water. Water was restricted from 1 h pre-dose to 1 h post-dose, and all food was forbidden within 4 h after dosing. Rescue food supplementation was allowed as needed for subjects experiencing hypoglycemia.

### 1.4 Investigational drug

Test formulation (T): Formula 3, specification: 15 mg; administered dose: 90 mg.

### 1.5 Pharmacokinetic results

**Table 5**

| | t_{1/2} (h) | Cₘₐₓ (ng/mL) | AUC₀₋ₜ (h*ng/mL) | AUC_{0-∞} (h*ng/mL) | Tₘₐₓ (h) |
|---|---|---|---|---|---|
| Fasted | 7.44 | 1370±548 | 22700±3382 | 23200±3387 | 6 |
| Fed | 7.89 | 810±280 | 17900±4439 | 20500±2112 | 12 |

### Example 3

In accordance with the doses listed in Table 6, 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid (compound A), microcrystalline cellulose, mannitol, povidone, anhydrous sodium carbonate and magnesium stearate were blended, followed by dry granulation and sizing. The sized granules were then homogeneously blended with croscarmellose sodium and magnesium stearate. The resulting final blended granules were compressed into tablets.

**Table 6**

| Ingredient | Formula 4 | Formula 5 | Formula 6 | Formula 7 |
|---|---|---|---|---|
| | mg/tablet | mg/tablet | mg/tablet | mg/tablet |
| Compound A | 60.0 | 60.0 | 60.0 | 60.0 |
| Microcrystalline cellulose 112 | 217.0 | 217.0 | 217.0 | 217.0 |
| Mannitol 200SD | 217.0 | 217.0 | 217.0 | 217.0 |
| Povidone K30 | 11.0 | 11.0 | 11.0 | 11.0 |
| Anhydrous sodium carbonate | 11.0 | 45.0 | 22.0 | 22.0 |
| Magnesium stearate | 4.5 | 4.5 | 4.5 | 4.5 |
| Croscarmellose sodium (additionally added) | 26.0 | 26.0 | 13.0 | 40.0 |
| Magnesium stearate (additionally added) | 5.0 | 5.0 | 5.0 | 5.0 |
| Total | 551.5 | 585.5 | 549.5 | 576.5 |

### Dissolution test

Dissolution tests of tablets from formula 4, formula 5, formula 6 and formula 7 were performed in accordance with the Second Method (Paddle Method) for dissolution measurement specified in Appendix of Volume II of Chinese Pharmacopoeia 2020 Edition. A total of 1000 ml of pH 5.0 acetate buffer containing 0.2% cetyltrimethylammonium bromide (CTAB) was adopted as dissolution medium, and the test was conducted at a paddle rotation speed of 50 rpm under 37±0.5 °C.

**Table 7**

| | Formula 4 | | Formula 5 | | Formula 6 | | Formula 7 | |
|---|---|---|---|---|---|---|---|---|
| Time (h) | Dissolution % | RSD% | Dissolution % | RSD% | Dissolution % | RSD% | Dissolution % | RSD% |
| 1 | 55 | 11.2 | 25 | 5.4 | 40 | 5.6 | 48 | 15.4 |
| 2 | 65 | 8.6 | 35 | 5.6 | 48 | 5.8 | 58 | 7.4 |
| 4 | 79 | 2.5 | 54 | 8.9 | 65 | 4.8 | 74 | 2.0 |
| 6 | 85 | 1.8 | 58 | 2.5 | 75 | 8.2 | 81 | 1.3 |
| 8 | 95 | 2.2 | 67 | 3.8 | 80 | 3.1 | 87 | 1.3 |
| 12 | 99 | 2.0 | 74 | 4.8 | 85 | 1.2 | 93 | 0.5 |

### Example 4

In accordance with the doses listed in Table 8, 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid (compound A), microcrystalline cellulose, mannitol, povidone, anhydrous sodium carbonate or calcium carbonate and magnesium stearate were blended, followed by dry granulation and sizing. The sized granules were then homogeneously blended with croscarmellose sodium and magnesium stearate. The resulting final blended granules were compressed into tablets.

**Table 8**

| Ingredient | Formula 8 | Formula 9 |
|---|---|---|
| | mg/tablet | mg/tablet |
| Compound A | 60.0 | 60.0 |
| Microcrystalline cellulose 112 | 217.0 | 217.0 |
| Mannitol 200SD | 217.0 | 217.0 |
| Povidone K30 | 11.0 | 11.0 |
| Anhydrous sodium carbonate | 22.0 | / |
| Calcium carbonate | / | 22.0 |
| Magnesium stearate | 4.5 | 4.5 |
| Croscarmellose sodium (additionally added) | 26.0 | 26.0 |
| Magnesium stearate (additionally added) | 5.0 | 5.0 |

### Dissolution test

Dissolution tests of tablets from formula 1 and formula 2 were performed in accordance with the Second Method (Paddle Method) for dissolution measurement specified in Appendix of Volume II of Chinese Pharmacopoeia 2020 Edition. A total of 1000 ml of pH 5.0 acetate buffer containing 0.2% cetyltrimethylammonium bromide (CTAB) was adopted as dissolution medium, and the test was conducted at a paddle rotation speed of 50 rpm under 37±0.5 °C.

**Table 9**

| | Formula 8 | | Formula 9 | |
|---|---|---|---|---|
| Time (h) | Dissolution % | RSD% | Dissolution % | RSD% |
| 1 | 43 | 5.6 | 99 | 0.5 |
| 2 | 55 | 7.4 | 99 | 0.8 |
| 4 | 65 | 2.0 | 100 | 0.1 |
| 6 | 70 | 1.3 | / | / |
| 8 | 80 | 1.3 | / | / |
| 12 | 92 | 0.5 | / | / |

| | | | | |
|---|---|---|---|---|
| Note: "/" indicates no sample tested | | | | |

The results show that compared with water-soluble sodium carbonate, the formulas containing calcium carbonate achieved almost complete dissolution within 1 h and exhibited no sustained-release properties.

### Test Example 2:

### 1.1 Study design

Twelve healthy adult male subjects were randomly divided into two groups, with 6 subjects per group. Blood samples were collected at scheduled time points, and safety assessments were performed. Plasma drug concentrations at various post-dose time points were quantified via HPLC-MS/MS. Pharmacokinetic parameters were calculated and subjected to statistical analysis.

### 1.2 Dosing regimen

All subjects were admitted to the clinical trial ward on the afternoon one day prior to the trial. A standard light dinner was provided in the evening, followed by a 10-h overnight fast with water permitted. Subjects took a standard breakfast at approximately 7:30 a.m., and the study drug was administered at around 8:00 a.m. with 240 mL of water. Physicians were required to examine the oral cavity to confirm complete drug intake. Water intake was allowed as needed except within 1 h before and after dosing. A standard meal was served 4 h post-dose (about 12:00). Subjects were required to stay in the ward for 24 h after administration for observation, and only standard meals supplied by the hospital were allowed during hospitalization. Throughout the trial, smoking, alcohol, coffee and carbonated drinks were prohibited. Pitaya, grapefruit, mango and their corresponding fruit juices were forbidden. Strenuous exercise and prolonged bed rest were to be avoided. The washout period between dosing cycles was 72 h (3 days).

Blood sampling time points: A 4 mL blood sample was collected at pre-dose (0 h), as well as 1 h, 2 h, 4 h, 6 h, 10 h, 12 h, 14 h, 16 h, 24 h, 30 h and 48 h after drug administration.

Blood samples were anticoagulated with sodium heparin, and the actual sampling time was recorded precisely. Samples were centrifuged at 3500 rpm for 10 min to separate plasma, which was stored at -80 °C until analysis.

### 1.3 Investigational drug

Test formulations (T): Formula 8 and formula 9, dose: 60 mg.

### 1.4 Pharmacokinetic results

**Table 10**

| | Formula 8 | Formula 9 |
|---|---|---|
| Tₘₐₓ (hr) | 11.2 | 3.1 |
| Cₘₐₓ (ng/mL) | 480 | 803 |
| T_{1/2} (hr) | 6.81 | 6.15 |
| AUCₗₐₛₜ (hr*ng/mL) | 9437 | 11291 |
| AUC_{INF_}pred (hr*ng/mL) | 9564 | 11371 |
| MRTₗₐₛₜ (h) | 15 | 11 |

In the formula 9 group, dizziness was reported in 1 subject, nausea in 6 subjects and vomiting in 5 subjects. Compared with the sustained-release formula 8 group, formula 9 presented 5 cases of vomiting with more prominent gastrointestinal adverse reactions.

### Example 5

According to the doses listed in Table 11, 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid (compound A), microcrystalline cellulose, mannitol, povidone, alkaline excipients (sodium carbonate, calcium hydroxide, meglumine or tris(hydroxymethyl)aminomethane) and croscarmellose sodium were blended, followed by the addition of magnesium stearate for further mixing. The resulting mixture was compressed into tablets.

**Table 11**

| Ingredient | Formula 10 | Formula 11 | Formula 12 | Formula 13 |
|---|---|---|---|---|
| | mg/tablet | mg/tablet | mg/tablet | mg/tablet |
| Compound A | 30.0 | 30.0 | 30.0 | 30.0 |
| Microcrystalline cellulose | 112.4 | 112.4 | 112.4 | 112.4 |
| Mannitol | 112.4 | 112.4 | 112.4 | 112.4 |
| Povidone | 5.6 | 5.6 | 5.6 | 5.6 |
| Sodium carbonate | 11.4 | / | / | / |
| Calcium hydroxide | / | 11.4 | / | / |
| Meglumine | / | / | 11.4 | / |
| Tris(hydroxymethyl)ami nomethane | / | / | / | 11.4 |
| Croscarmellose sodium | 5.6 | 5.64 | 5.64 | 5.64 |
| Magnesium stearate | 4.9 | 4.9 | 4.9 | 4.9 |

| | | | | |
|---|---|---|---|---|
| Note: "/" indicates no testing | | | | |

Dissolution method: The dissolution test was performed in accordance with the First Method (Basket Method) for dissolution determination specified in the Appendix of Volume II of the Chinese Pharmacopoeia 2020 Edition. A total of 900 ml of pH 5.0 acetate buffer solution containing 0.2% cetyltrimethylammonium bromide (CTAB) was used as the dissolution medium. The test was conducted at 37±0.5 °C with a rotation speed of 100 rpm.

**Table 12**

| Dissolution time | Formula 10 | Formula 11 | Formula 12 | Formula 13 |
|---|---|---|---|---|
| | Dissolution % | Dissolution % | Dissolution % | Dissolution % |
| 1h | 45 | 34 | 87 | 91 |
| 2h | 52 | 72 | 99 | 93 |
| 4h | 58 | 95 | 100 | 93 |
| 6 h | 65 | 98 | 100 | 94 |
| 8 h | 70 | 98 | 100 | 94 |
| 12 h | 77 | 98 | 100 | 95 |

The above results indicate that different types of alkaline excipients exert distinct effects on dissolution and release.

## Claims

1. A pharmaceutical composition, comprising a compound represented by formula I or a pharmaceutically acceptable salt thereof as an active ingredient, and an acid-resistant compound, 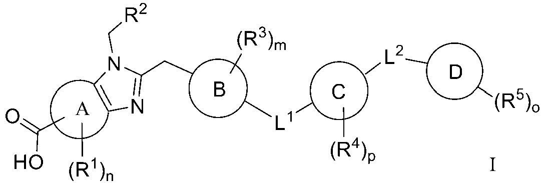 wherein,
ring A is selected from phenyl or 5- or 6-membered heteroaryl;
each R¹ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
R² is selected from 3- to 6-membered heterocycloalkyl or 5- or 6-membered heteroaryl, wherein the heterocycloalkyl or heteroaryl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
ring B is selected from 3- to 6-membered heterocycloalkyl, phenyl, or 5- or 6-membered heteroaryl;
each R³ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
or any two R³ groups, together with the adjacent atoms to which they are attached, form C₃₋₆ cycloalkyl or 3- to 6-membered heterocycloalkyl, wherein the cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
L¹ is selected from -O-, -S-, -C(O)O-, -OC(O)-, -C(O)-, -S(O)-, -S(O)₂-, -C(R^{1a}R^{1b})-, -OC(R^{1a}R^{1b})-, -C(R^{1a}R^{1b})O-, -C(O)N(R^{1c})-, -N(R^{1c})(O)C-, -S(O)₂N(R^{1d})-, -N(R^{1e})- or a bond,
each of R^{1a}, R^{1b}, R^{1c}, R^{1d} and R^{1e} is independently selected from hydrogen, halogen, hydroxy, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl or C₁₋₆ alkoxy;
ring C is selected from C₆₋₁₀ aryl or 5- or 10-membered heteroaryl;
each R⁴ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
L² is selected from-O-, -S-, -C(O)O-, -OC(O)-, -C(O)-, -S(O)-, -S(O)₂-, -C(R^{2a}R^{2b})-, -OC(R^{2a}R^{2b})-, -C(R^{2a}R^{2b})O-, -C(O)N(R^{2c})-, -N(R^{2c})(O)C-, -S(O)₂N(R^{2d})-, -N(R^{2e})- or a bond;
each of R^{2a}, R^{2b}, R^{2c}, R^{2d} and R^{2e} is independently selected from hydrogen, halogen, hydroxy, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl or C₁₋₆ alkoxy;
ring D is selected from C₆₋₁₀ aryl or 5- or 10-membered heteroaryl;
each R⁵ is the same or different and is independently selected from halogen, hydroxy, cyano, nitro, amino, C₁₋₆ alkyl, C₁₋₆ alkoxy, C₃₋₆ cycloalkyl or 3- to 6-membered heterocycloalkyl, wherein the alkyl, alkoxy, cycloalkyl or heterocycloalkyl is optionally substituted with one or more groups selected from halogen, hydroxy, cyano, nitro, amino, C₁₋₆ alkyl, C₁₋₆ alkoxy, C₃₋₆ cycloalkyl or 3- to 6-membered heterocycloalkyl;
n is selected from 0, 1, 2, 3 or 4;
m is selected from 0, 1, 2, 3 or 4;
o is selected from 0, 1, 2, 3 or 4;
p is selected from 0, 1, 2, 3 or 4.

2. The pharmaceutical composition according to claim 1, wherein the acid-resistant compound is a water-soluble acid-resistant compound, preferably sodium carbonate, sodium bicarbonate, potassium carbonate, or potassium bicarbonate.

3. The pharmaceutical composition according to claim 1 or 2, further comprising a pharmaceutically acceptable excipient, wherein the excipient is preferably at least one selected from fillers, disintegrants, binders, and lubricants.

4. The pharmaceutical composition according to any one of claims 1 to 3, wherein the compound represented by formula I or a pharmaceutically acceptable salt thereof is a compound represented by formula II-1 or a pharmaceutically acceptable salt thereof, 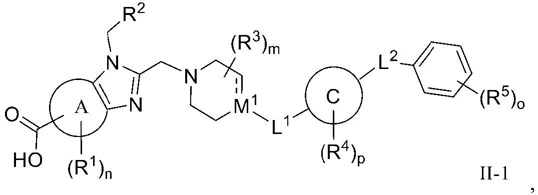 wherein M¹ is an N atom or a C atom; - - - - - is a single bond or a double bond; when M is an N atom, - - - - - is a single bond; when M is a C atom, - - - - - is a single bond or a double bond; ring A, ring C, R¹, R², R³, R⁴, R⁵, L¹, L², n, m, o and p are as defined in claim 1.

5. The pharmaceutical composition according to claim 1 or 4, wherein ring A is selected from phenyl or 5-membered heteroaryl, preferably

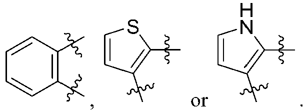

6. The pharmaceutical composition according to claim 1 or 4, wherein ring C is selected from 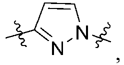 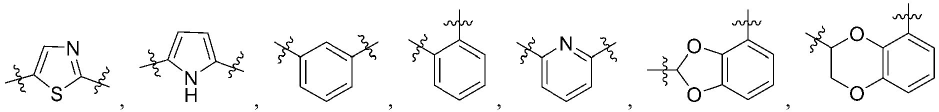 or

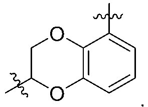

7. The pharmaceutical composition according to claim 1 or 4, wherein the compound represented by formula I or a pharmaceutically acceptable salt thereof is selected from a compound represented by formula II-1a or a pharmaceutically acceptable salt thereof, a compound represented by formula II-1b or a pharmaceutically acceptable salt thereof, a compound represented by formula II-1c or a pharmaceutically acceptable salt thereof, or a compound represented by formula II-1d or a pharmaceutically acceptable salt thereof, or a compound represented by formula II-1e or a pharmaceutically acceptable salt thereof, or a compound represented by formula II-1f or a pharmaceutically acceptable salt thereof, 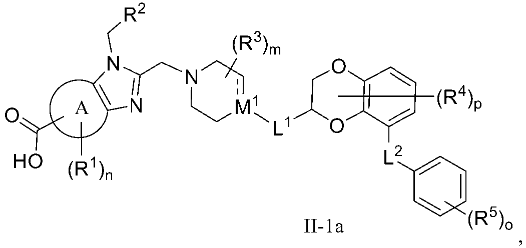 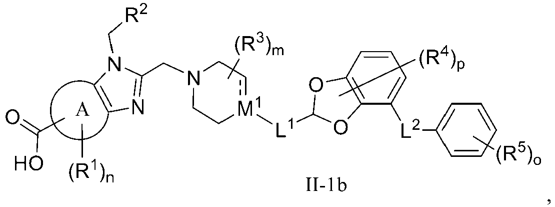 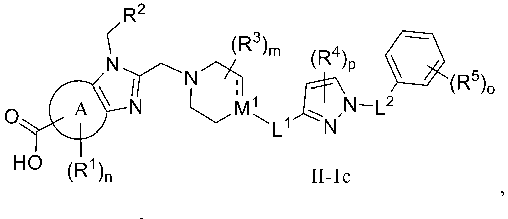 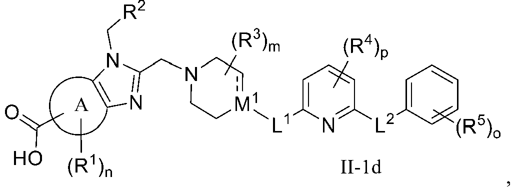 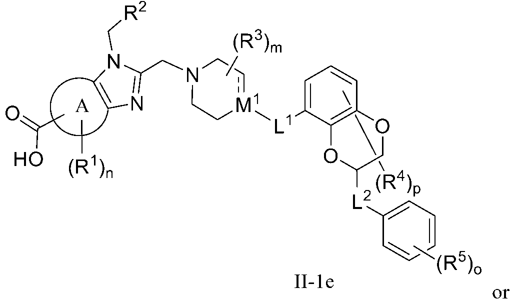 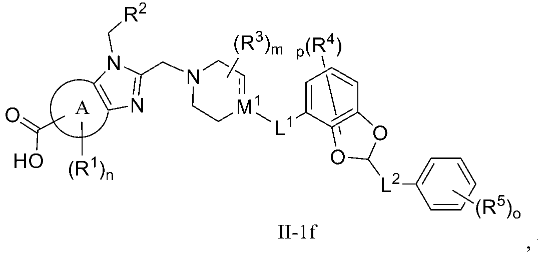 wherein ring A, R¹, R², R³, R⁴, R⁵, L¹, L², n, m, o and p are as defined in claim 1; - - - - - is a single bond or a double bond; and M¹ is as defined in claim 7.

8. The pharmaceutical composition according to claim 1, 4 or 7, wherein L¹ is selected from a bond, and L² is selected from a bond.

9. The pharmaceutical composition according to claim 1, 4 or 7, wherein each R⁵ is the same or different and is independently selected from halogen, cyano, C₁₋₆ alkyl or 3- to 6-membered heterocycloalkyl, wherein the heterocycloalkyl is optionally substituted with one or more groups selected from halogen, hydroxy, cyano, nitro, amino, C₁₋₆ alkyl or C₁₋₆ alkoxy; or each R⁵ is the same or different and is independently selected from halogen, cyano, C₁₋₆ alkyl or C₁₋₆ alkoxy, wherein the alkyl or alkoxy is optionally substituted with one or more groups selected from halogen, hydroxy, cyano, nitro, amino, C₁₋₆ alkyl or C₁₋₆ alkoxy.

10. The pharmaceutical composition according to claim 1, wherein the compound represented by formula I or a pharmaceutically acceptable salt thereof is selected from:

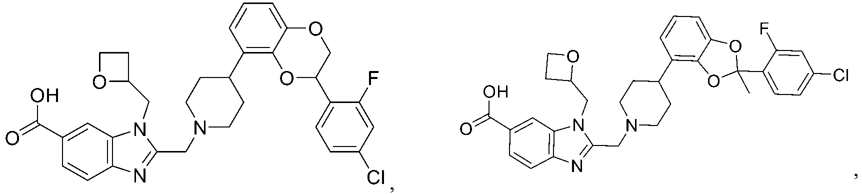

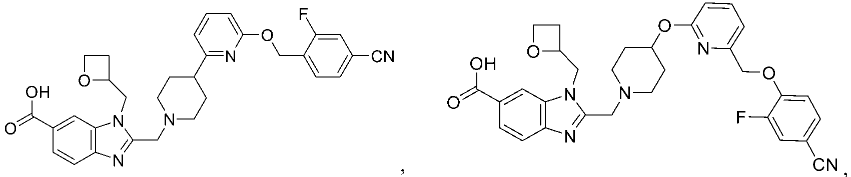

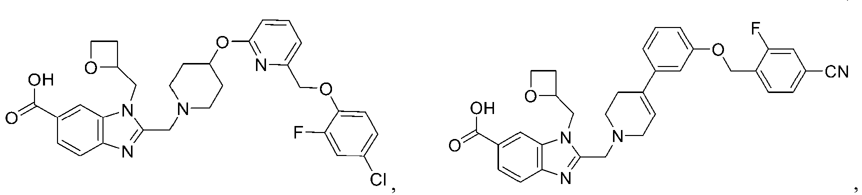

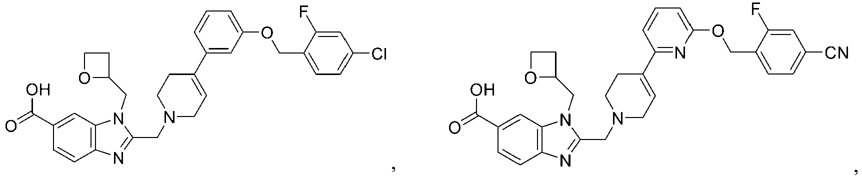

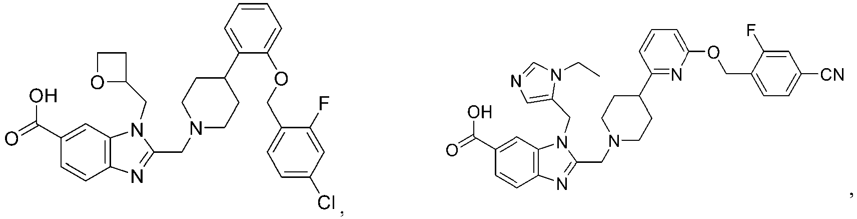

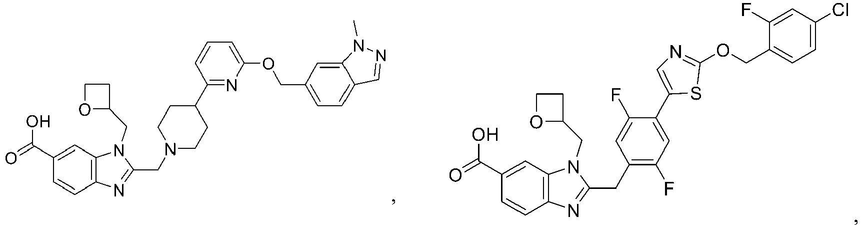

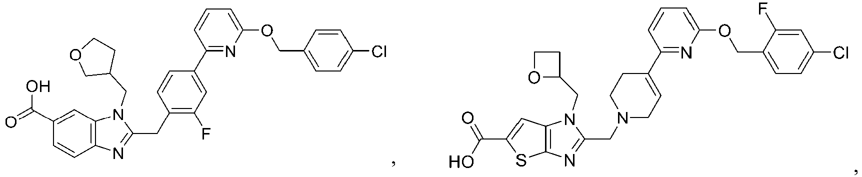

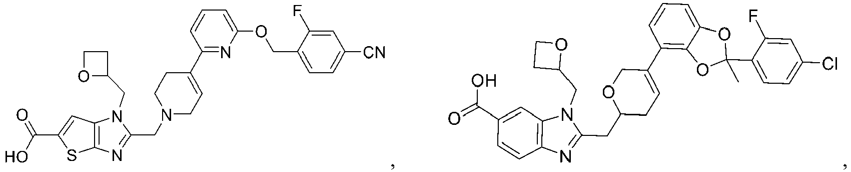

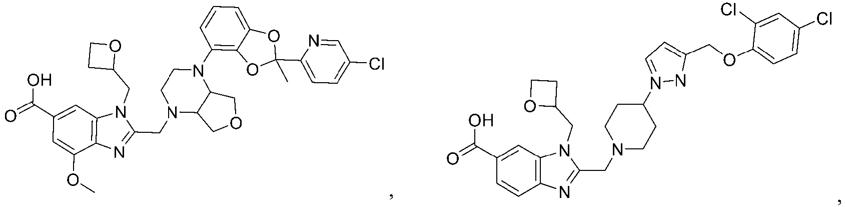

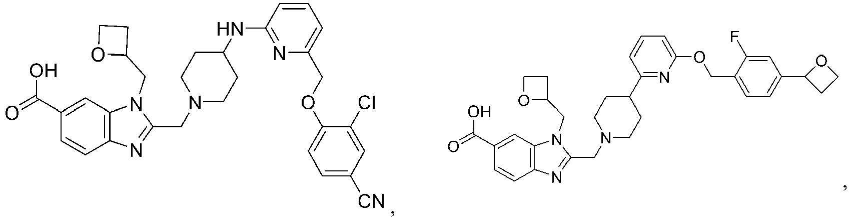

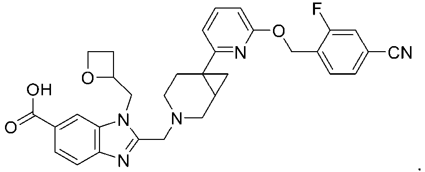

11. The pharmaceutical composition according to claim 3, wherein the filler is selected from at least one of lactose, sucrose, starch, pregelatinized starch, mannitol, dibasic calcium phosphate and microcrystalline cellulose, and preferably, the filler accounts for 20-95% by weight of the pharmaceutical composition;
the disintegrant is selected from at least one of croscarmellose sodium, crospovidone, sodium starch glycolate and low-substituted hydroxypropyl cellulose, and preferably, the disintegrant accounts for 1-20% by weight of the pharmaceutical composition;
the binder is selected from at least one of povidone, copovidone, starch, methylcellulose, carboxymethylcellulose, hydroxypropyl cellulose, hydroxypropyl methylcellulose and alginate; preferably, the binder accounts for 0.5-10% by weight of the pharmaceutical composition;
the lubricant is selected from at least one of magnesium stearate, stearic acid, palmitic acid, talc and sodium stearyl fumarate, and preferably, the lubricant accounts for 0.1-5% by weight of the pharmaceutical composition.

12. The pharmaceutical composition according to claim 1 or 11, comprising:
1) 1%-30% of 2-((4-((S)-3-(4-chloro-2-fluorophenyl)-2,3-dihydrobenzo[b][1,4]dioxan-5-yl)piperidin-1-yl)methyl)-1-(((S)-oxetan-2-yl)methyl)-1H-benzo[d]imidazole-6-carboxylic acid or a pharmaceutically acceptable salt thereof as an active ingredient;
2) 20-95% of a filler;
3) 0.5-10% of an acid-resistant compound;
4) 1-20% of a disintegrant; and 5) 0.5-10% of a binder.

13. The pharmaceutical composition according to claim 2, wherein the weight ratio of the acid-resistant compound to the active ingredient is 1:1 to 1:40, preferably 1:1 to 1:10, for example 1:1, 1:2, 1:3 or 1:4.

14. The pharmaceutical composition according to claim 1, wherein oral administration of the compound represented by formula I or a pharmaceutically acceptable salt thereof at a dose of 90 mg to a population of healthy fed subjects provides a mean plasma Cₘₐₓ of 810±280 ng/mL.

15. The pharmaceutical composition according to claim 1, wherein oral administration of the compound represented by formula I or a pharmaceutically acceptable salt thereof to a population of healthy fed subjects provides a Tₘₐₓ of at least 7 h.

16. The pharmaceutical composition according to claim 1, wherein oral administration of the compound represented by formula I or a pharmaceutically acceptable salt thereof to a population of healthy subjects provides a T_{1/2} ranging from 6 h to 8 h.

17. Use of the pharmaceutical composition according to any one of claims 1 to 16 in the manufacture of a drug for preventing and/or treating Type I diabetes mellitus, Type II diabetes mellitus, malnutrition-related diabetes mellitus, diabetic complications, obesity, hyperglycemia, impaired glucose tolerance, cardiovascular diseases, hyperlipidemia, cerebral infarction, stroke, non-alcoholic steatohepatitis (NASH), Parkinson's disease, dementia, insulin resistance and hepatic insulin resistance; preferably, use in the manufacture of a drug for preventing and/or treating Type I diabetes mellitus, Type II diabetes mellitus, obesity, diabetic complications, non-alcoholic steatohepatitis and cardiovascular diseases.
